(19) Europäisches Patentamt
European Patent Office
Office européen des brevets

(11) EP 2 763 656 B1

(12) FASCICULE DE BREVET EUROPEEN

(45) Date de publication et mention de la délivrance du brevet:
**07.12.2016 Bulletin 2016/49**

(21) Numéro de dépôt: **12766078.5**

(22) Date de dépôt: **26.09.2012**

(51) Int Cl.:
*A61K 49/18* (2006.01) *A61K 9/107* (2006.01)
*A61K 47/28* (2006.01) *G01N 33/574* (2006.01)
*G01N 33/48* (2006.01) *G01N 33/74* (2006.01)
*C12Q 1/68* (2006.01) *A61K 47/10* (2006.01)
*A61K 47/24* (2006.01) *A61K 47/44* (2006.01)
*A61K 51/12* (2006.01) *A61K 31/138* (2006.01)
*A61K 31/565* (2006.01) *A61K 31/57* (2006.01)
*G01N 33/50* (2006.01) *A61K 31/575* (2006.01)

(86) Numéro de dépôt international:
**PCT/EP2012/068944**

(87) Numéro de publication internationale:
**WO 2013/050280 (11.04.2013 Gazette 2013/15)**

(54) **FORMULATIONS POUR LE DIAGNOSTIC ET LE TRAITEMENT DE CANCERS HORMONODÉPENDANTS ET DE CANCERS DES ORGANES DE SYNTHÈSE D'HORMONES STÉROÏDIENNES.**

FORMULIERUNGEN ZUR DIAGNOSE UND BEHANDLUNG VON HORMONABHÄNGIGEN KREBSARTEN UND KREBS DER FÜR DIE STEROIDHORMONSYNTHESE VERANTWORTLICHEN ORGANE

FORMULAS FOR DIAGNOSING AND TREATING HORMONE-DEPENDENT CANCERS AND CANCERS OF THE ORGANS RESPONSIBLE FOR STEROID HORMONE SYNTHESIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.10.2011 FR 1158991**

(43) Date de publication de la demande:
**13.08.2014 Bulletin 2014/33**

(73) Titulaire: **Commissariat à l'Énergie Atomique et aux Énergies Alternatives**
**75015 Paris (FR)**

(72) Inventeurs:
- **MERIAN, Juliette**
**F-19100 Brive (FR)**
- **BOISGARD, Raphaël**
**F-91620 Nozay (FR)**
- **TAVITIAN, Bertrand**
**F-75015 Paris (FR)**
- **TEXIER-NOGUES, Isabelle**
**F-38000 Grenoble (FR)**

(74) Mandataire: **Lavoix**
**2, place d'Estienne d'Orves**
**75441 Paris Cedex 09 (FR)**

(56) Documents cités:
**FR-A1- 2 956 320**

- **ALMEIDA CRISTINA P ET AL: "Modification of composition of a nanoemulsion with different cholesteryl ester molecular species: effects on stability, peroxidation, and cell uptake.", INTERNATIONAL JOURNAL OF NANOMEDICINE 2010 LNKD- PUBMED:20957219, vol. 5, 2010, pages 679-686, XP002670190, ISSN: 1178-2013**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne une formulation de type nanoémulsion, un procédé pour sa préparation ainsi que son utilisation pour le traitement ou le diagnostic de cancers hormonodépendants ou de cancers des organes de synthèse d'hormones stéroïdiennes.

### [**Etat de la technique**]

La nanomédecine constitue un champ nouveau créé par la fusion de la nanotechnologie et de la médecine, et est aujourd'hui l'une des voies les plus prometteuses pour le développement de thérapies ciblées efficaces ou de nouvelles méthodes de diagnostic.
A cet effet, les demandes internationales WO 2010/018223 et WO 2008/102065 décrivent des formulations sous forme de nanoémulsions, comprenant une phase aqueuse continue et au moins une phase huileuse dispersée, dans laquelle :

- la phase huileuse comprend :

  - au moins un agent thérapeutique (WO 2010/018223) ou de diagnostic (WO 2008/102065),
  - au moins un lipide amphiphile, et
  - au moins un lipide solubilisant, et

- la phase aqueuse comporte au moins un co-tensioactif comprenant au moins une chaîne composée de motifs d'oxyde d'alkylène.

Ces formulations sont utiles pour la délivrance d'agents thérapeutiques lipophiles ou amphiphiles ou pour l'imagerie optique.
Par ailleurs, le cancer est la seconde cause de mortalité dans le monde. En France, on estime qu'une personne sur trois meurt d'un cancer au cours de sa vie. Parmi les plus rependus, les cancers hormonodépendants, pour lesquels la prolifération cellulaire dépend de la présence d'hormones : principalement l'oestrogène pour le cancer du sein et majoritairement l'androgène pour le cancer de la prostate. D'autre part, les organes de synthèse d'hormones stéroïdiennes, tels que les ovaires ou les surrénales, peuvent aussi être affectés par des cancers, souvent détectés très tardivement et difficiles à soigner.
Almeida et al. (Int. J. Nanomedicine, 5, 2010, 679-686) décrit des nanoémulsions comprenant de l'oléate, du stéarate ou du linoléate de cholestéryle, de la phosphotidylcholine d'oeuf et du cholestérol, et de la trioléine. Cet article enseigne que des nanoémulsions riches en ester de cholestéryle peuvent être utilisées comme système de délivrance pour cibler notamment les tumeurs. Cela étant, cet article ne suggère aucunement que les nanoémulsions décrites peuvent cibler préférentiellement les cancers hormonodépendants par rapport aux cancers non-hormonodépendants. De plus, les phases dispersées des nanoémulsions comprennent de 25 à 31% en poids d'esters de cholestéryle. Or, les inventeurs de la présente demande ont montré que leurs nanoémulsions (décrites notamment dans les demandes internationales WO 2010/018223 et WO 2008/102065) ne pouvaient pas être préparées avec de telles proportions d'esters de cholestéryle. Le développement de nouvelles méthodes de diagnostic et de traitement des cancers hormonodépendants et des cancers des organes de synthèse d'hormones stéroïdiennes est nécessaire.

### [Problème technique]

A cet effet, la présente invention fournit une formulation qui cible préférentiellement les cancers hormonodépendants et les cancers des organes de synthèse d'hormones stéroïdiennes. Ainsi, elle est utile pour le diagnostic ou pour le traitement de cancers hormonodépendants ou de cancers des organes de synthèse d'hormones stéroïdiennes.

### [Description de l'invention]

### [Définitions]

Dans le cadre de cet exposé, on entend par le terme « nanoémulsion » une composition présentant au moins deux phases, généralement une phase huileuse et une phase aqueuse, dans laquelle la taille moyenne de la phase dispersée est inférieure à 1 micron, de préférence de 10 à 500 nm et en particulier de 20 à 100 nm, et tout préférentiellement 20 et 70 nm (voir article C. Solans, P. Izquierdo, J. Nolla, N. Azemar and M. J. Garcia-Celma, Curr Opin Colloid In, 2005, 10, 102-110).

Le terme « gouttelette » englobe à la fois les gouttelettes d'huile liquide proprement dites ainsi que les particules solides issues d'émulsions de type huile-dans-eau dans lesquelles la phase huileuse est solide. Dans ce dernier cas, on parle souvent aussi d'émulsion solide.

Au sens de la présente demande, l'expression « phase huileuse dispersée » également appelée « phase dispersée » ou « phase huileuse », signifie les gouttelettes comprenant l'éventuelle huile/ le lipide solubilisant/ le lipide amphiphile/ le co-tensioactif (éventuellement greffé par une molécule d'intérêt ou par un agent de diagnostic ou thérapeutique)/ le composé de formule (I)/ l'éventuel agent thérapeutique / l'éventuel agent de diagnostic. En particulier, pour les calculs de proportion massique de composé de formule (I) par rapport au poids de la phase dispersée, on considère que le co-tensioactif appartient à la phase dispersée. La phase dispersée est généralement exempte de phase aqueuse.

Le terme « lipide » désigne dans le cadre de cet exposé l'ensemble des corps gras ou des substances contenant des acides gras présents dans les graisses d'origine animales et dans les huiles végétales. Ce sont des molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on citera en particulier la phosphatidylcholine, la phosphatidyl éthanolamine, la phophatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un tensioactif pour abaisser davantage l'énergie de l'interface.

On entend par le terme « alkyle » un groupe aliphatique hydrocarboné, saturé, linéaire ou ramifié. Le groupe alkyle est de préférence linéaire. On entend par le terme « alcényle » un groupe aliphatique hydrocarboné comprenant au moins une insaturation sous forme de double liaison, linéaire ou ramifié, de préférence linéaire.

Au sens de la présente demande, on entend par le terme « stéroïdique» un radical dérivé d'un stéroïde par la perte d'un hydrogène sur l'un des atomes d'un des cycles du stéroïde. On entend par le terme « stérolique» un radical dérivé d'un stérol par la perte d'un hydrogène sur l'un des atomes d'un des cycles du stéroïde. Au sens de la présente demande, les stéroïdes et stérols ont la définition commune de l'IUPAC (International Union of Pure and Applied Chemistry) (Pure&Appl. Chem.,Vol. 61, No. 10, pp. 1783-1822,1989). Un stéroïde est un composé comprenant un noyau cyclopenta[*a*]phenanthrenique (stérane) partiellement ou complètement hydrogéné ou un squelette dérivé de celui-ci par la scission d'une ou plus liaisons ou par des contractions ou expansions de cycles. Les stéroïdes préférés sont ceux comprenant un noyau cyclopenta[*a*]phenanthrenique (stérane) partiellement ou complètement hydrogéné. Un stérol est un stéroïde comprenant un groupe hydroxyle en position C3.

**[Emulsion]**

Selon un premier aspect, l'invention concerne une formulation d'un agent thérapeutique ou de diagnostic sous forme de nanoémulsion, comprenant une phase aqueuse continue et au moins une phase huileuse dispersée, dans laquelle :

- la phase huileuse comprend :
- au moins un lipide amphiphile,
- au moins un lipide solubilisant,
- au moins un composé de formule (I) suivante :

$$(R\text{-}L)_n\text{-}A \qquad (I),$$

dans laquelle :

- n représente un nombre entier de 1 à 5, notamment 1 à 2, de préférence 1,
- L représente une liaison simple ou un groupe divalent choisi parmi -O- , -COO-, -OOC-, -CO-NR'-, -NR'-CO-, -S-, -NR'-CO-NR"-, -O-CO-O-, où R' et R" représentent indépendamment H ou un alkyle linéaire ou ramifié de 1 à 20 atomes de carbone et
- R représente un alkyle linéaire ou ramifié comprenant au moins 11 atomes de carbone ou un alcényle linéaire ou ramifié comprenant au moins 11 atomes de carbone, et
- A représente un groupe stéroïdique ou stérolique,
- la phase aqueuse comporte au moins un co-tensioactif comprenant au moins une chaîne composée de motifs d'oxyde d'alkylène.

L'émulsion est donc une émulsion de type huile dans l'eau. Elle peut être simple ou multiple, notamment en comportant dans la phase dispersée une seconde phase aqueuse. De préférence, elle est simple.

• Composé de formule (I)

L'émulsion comprend au moins un composé comprenant un groupe A stéroïdique ou stérolique lié par au moins un groupe L à une chaîne hydrocarbonée comprenant au moins 11 atomes de carbone (groupe R).

Dans la formulation selon l'invention, le composé de formule (I) est notamment utile en tant qu'agent de diagnostic ou d'agent thérapeutique pour le traitement de cancers hormonodépendants ou de cancers des organes de synthèse d'hormones stéroïdiennes.

Dans un mode de réalisation, le groupe A est un groupe ayant l'une des formules suivantes :

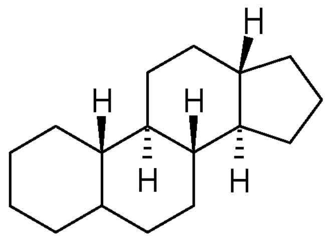

(IIa)

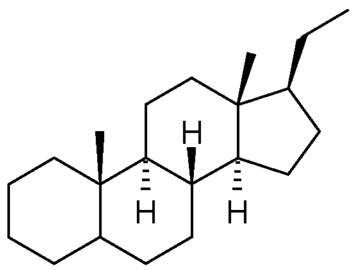

(IIb),

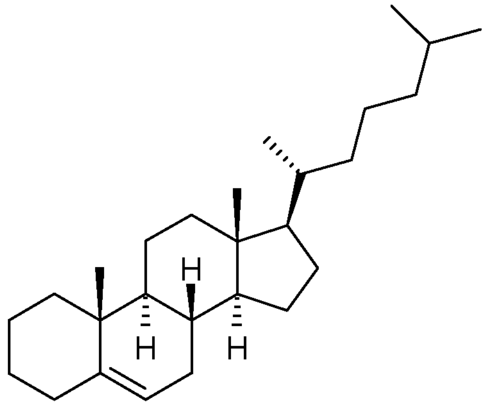

(IIc)

dans laquelle un ou plusieurs des atomes de carbone est substitué par un groupe -L-R.

Dans un mode de réalisation préféré, le groupe A est substitué en position C3 par un groupe -L-R. Est particulièrement préféré le composé de formule (III) suivante :

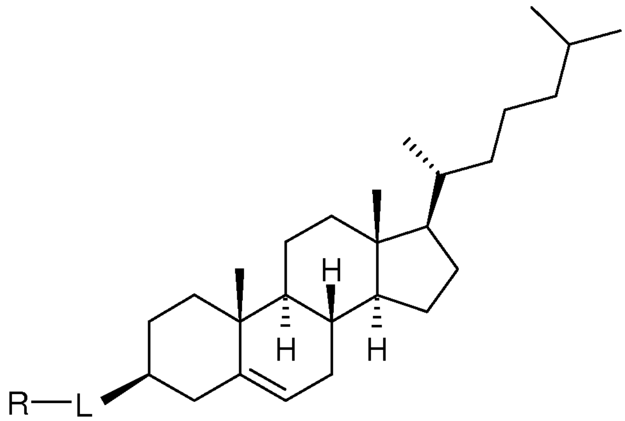

(III)

Lorsque L est un groupe -O- ou -COO-, le composé de formule (III) est respectivement un éther ou un ester de cholestéryle.

Le composé de formule (I) comprend n groupes -L-R.

De préférence, n représente 1, c'est-à-dire que le composé de formule (I) comprend un groupe stéroïdique ou stérolique substitué une fois par un groupe -L-R.

R représente un alkyle ou un alcényle comprenant au moins 11 atomes de carbone, notamment de 11 à 25 atomes de carbone, de préférence de 11 à 19 atomes de carbone.

L représente une liaison simple ou un groupe divalent choisi parmi -O- , -COO-, -OOC-, -CO-NR'-, -NR'-CO-, -S-, -NR'-CO-NR"-, -O-CO-O-. De préférence, L représente un groupe divalent. R' et R" représentent indépendamment H ou un alkyle linéaire ou ramifié de 1 à 20 atomes de carbone, notamment H ou un méthyle, de préférence H.

Dans un mode de réalisation préféré, L représente -O- ou -COO-. Lorsque L représente une fonction ester -COO-, il est sous-entendu que le groupe R est lié à l'atome de carbone de -COO- et le groupe A est lié à l'atome d'oxygène de -COO-.

L représente de préférence -COO-. Le composé a alors la formule (IV) :

$(R\text{-}COO)_n\text{-}A$ (IV)

où R, n et A sont tels que définis ci-dessus. De préférence, n représente 1 et le composé a la formule (V) suivante :

R-COO-A (V)

En effet, la fonction ester est avantageusement métabolisable, ce qui permet à l'organisme auquel la formulation selon l'invention est administrée d'éliminer le composé de formule (I) dans laquelle L représente -COO-. En comparaison, les

composés de formule (I) dans laquelle L représente -O- ont tendance à s'accumuler dans l'organisme.

De préférence, le composé a la formule (VI) suivante :

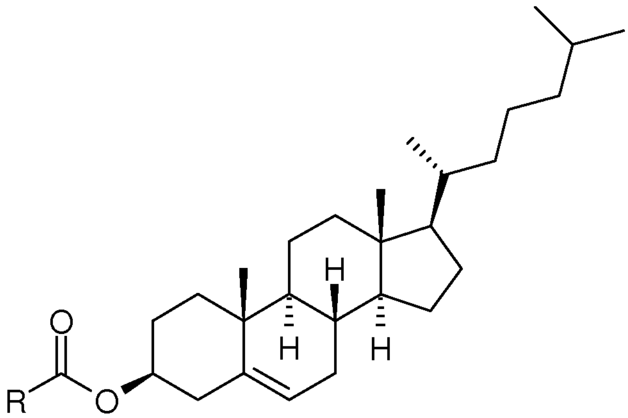

(VI)

dans laquelle R est tel que défini ci-dessus.

De préférence, le composé de formule (IV), (V) ou (VI) (formules dans lesquelles L représente -COO-) est un ester d'acide gras saturé ou insaturé, notamment choisi parmi les esters suivants :

| R | | Composé de formule (IV) ou (V) | Composé de formule (VI) | |
|---|---|---|---|---|
| alkyle | $CH_3(CH_2)_{10}$- | Laurate stéroïdique ou stérolique | Laurate de cholestéryle | ester d'acide gras saturé |
| | $CH_3(CH_2)_{12}$- | Myristate stéroïdique ou stérolique | Myristate de cholestéryle | |
| | $CH_3(CH_2)_{14}$- | Palmitate stéroïdique ou stérolique | Palmitate de cholestéryle | |
| | $CH_3(CH_2)_{16}$- | Stéarate stéroïdique ou stérolique | Stéarate de cholestéryle | |
| | $CH_3(CH_2)_{18}$- | Arachidate stéroïdique ou stérolique | Arachidate de cholestéryle | |
| | $CH_3(CH_2)_{20}$- | Béhénate stéroïdique ou stérolique | Béhénate de cholestéryle | |
| | $CH_3(CH_2)_{22}$- | Lignocérate stéroïdique ou stérolique | Lignocérate de cholestéryle | |
| | $CH_3(CH_2)_{24}$- | Cérotate stéroïdique ou stérolique | Cérotate de cholestéryle | |
| alcényle | $CH_3(CH_2)_3CH{=}CH(CH_2)_7$- | Myristoléate stéroïdique ou stérolique | Myristoléate de cholestéryle | Ester d'acide gras insaturé |
| | $CH_3(CH_2)_5CH{=}CH(CH_2)_7$- | Palmitoléate stéroïdique ou stérolique | Palmitoléate de cholestéryle | |
| | $CH_3(CH_2)_7CH{=}CH(CH_2)_7$- | Oléate stéroïdique ou stérolique | Oléate de cholestéryle | |
| | $CH_3(CH_2)_7CH{=}CH(CH_2)_7$- | Elaidate stéroïdique ou stérolique | Elaidate de cholestéryle | |

(suite)

| R | | Composé de formule (IV) ou (V) | Composé de formule (VI) | |
|---|---|---|---|---|
| alcényle | $CH_3(CH_2)_5CH{=}CH(CH_2)_9$- | Vaccénate stéroïdique ou stérolique | Vaccénate de cholestéryle | Ester d'acide gras insaturé |
| | $CH_3(CH_2)_4CH{=}CHCH_2CH{=}C\ H(CH_2)_7$- | Linoléate stéroïdique ou stérolique | Linoléate de cholestéryle | |
| | $CH_3(CH_2)_4CH{=}CHCH_2CH{=}C\ H(CH_2)_7$- | Linoelaidate stéroïdique ou stérolique | Linoelaidate de cholestéryle | |
| | $CH_3CH_2CH{=}CHCH_2CH{=}CHC\ H_2CH{=}CH(CH_2)_7$- | $\alpha$-Linolénate stéroïdique ou stérolique | $\alpha$-Linolénate de cholestéryle | |
| | $CH_3(CH_2)_4CH{=}CHCH_2CH{=}C\ HCH_2CH{=}CHCH_2$- $CH{=}CH(CH_2)_3$- | Arachidonat e stéroïdique ou stérolique | Arachidonate de cholestéryle | |
| | $CH_3CH_2CH{=}CHCH_2CH{=}CHC\ H_2CH{=}CHCH_2CH{=}CHCH_2CH\ {=}CH(CH_2)_3$- | Eicosapenta enate stéroïdique ou stérolique | Eicosapentae nate de cholestéryle | |
| | $CH_3(CH_2)_7CH{=}CH(CH_2)_{11}$- | Erucate stéroïdique ou stérolique | Erucate de cholestéryle | |
| | $CH_3CH_2CH{=}CHCH_2CH{=}CHC\ H_2CH{=}CHCH_2CH{=}CHCH_2CH\ {=}CHCH_2CH{=}CH(CH_2)_2$- | Docosahéxa énate stéroïdique ou stérolique | Docosahéxaé nate de cholestéryle | |

Parmi les composés de formule (I) dans lesquels L représente -COO-, le stéarate de cholestéryle et l'oléate de cholestéryle sont particulièrement préférés.

Parmi les composés de formule (I) dans lesquels L représente -O-, l'hexadécyléther de cholestéryle est préféré.

Le composé de formule (I) se localise majoritairement (à plus de 50%) dans la phase huileuse dispersée de la nanoémulsion, et minoritairement (moins de 50%) dans la phase aqueuse continue. Dans la présente demande, les pourcentages massiques de phase dispersée décrits sont calculés en considérant que le composé de formule (I) appartient à la phase dispersée.

Les composés de formules (I) sont disponibles dans le commerce ou peuvent être synthétisés par des méthodes connues de l'homme du métier. Par exemple, lorsque L représente -O- ou -COO- et A le cholestéryle, les composés peuvent être obtenus par éthérification ou par estérification du cholestérol.

Généralement, dans la formulation selon l'invention, la proportion de composé de formule (I) est entre 0,05 et 3% en poids, de préférence entre 0,3 et 2,5% en poids, par rapport au poids de la phase dispersée de la nanoémulsion (le poids du co-tensioactif étant inclus dans le poids de la phase dispersée). Plus précisément, le rapport de la masse de composé de formule (I) sur le l'ensemble des masses de (l'éventuelle huile/ le lipide solubilisant/ le lipide amphiphile/ le co-tensioactif (éventuellement greffé par une molécule d'intérêt ou par un agent de diagnostic ou thérapeutique)/ le composé de formule (I)/ l'éventuel agent thérapeutique / l'éventuel agent de diagnostic) est entre 0,05 et 3%. En effet, pour ces proportions, la taille des gouttelettes est généralement plus homogène, ce qui permet une libération plus homogène de l'agent thérapeutique ou de diagnostic. De plus, pour des proportions plus importantes, il n'est généralement pas possible de préparer la nanoémulsion. Sans vouloir être liés à une théorie particulière, il semblerait qu'au-delà de 3% en poids, le composé de formule (I) ne se solubilise pas majoritairement dans la phase huileuse dispersée de la nanoémulsion.

Dans un mode de réalisation préféré, la phase huileuse de la formulation selon l'invention comprend en outre au moins un agent thérapeutique pour le traitement de cancers hormonodépendants ou de cancers des organes de synthèse d'hormones stéroïdiennes, ou un agent de diagnostic (en plus du composé de formule (I)).

Avantageusement, comme l'agent thérapeutique ou de diagnostic est encapsulé dans les gouttelettes de la nanoémulsion, la formulation selon l'invention protège le corps dans lequel la formulation est administré dudit agent (celui-ci sera essentiellement situé au niveau du cancer hormonodépendant ou du cancer des organes de synthèse d'hormones stéroïdiennes, et peu ailleurs dans le corps) et protège également l'agent du corps (notamment en évitant

son métabolisme prématuré).

L'agent de diagnostic peut notamment être utilisé dans les imageries de type :

- tomographie à émission de positons (Positron Emission Tomography (PET) en anglais) (l'agent de diagnostic pouvant être un composé comprenant un radionucléide, tel $^{18}F$, $^{11}C$, un un chélate de cations métalliques $^{68}Ga$, $^{64}Cu$),
- tomographie d'émission monophotonique (TEMP) (Single photon emission computed tomography (SPECT) en anglais) (l'agent de diagnostic pouvant être un composé comprenant un radionucléide par exemple $^{123}I$, ou un chélate de $^{99m}Tc$ ou $^{111}In$),
- imagerie par résonance magnétique (IRM) (l'agent de diagnostic pouvant être un chélate de gadolinium ou un nanocristal magnétique tel qu'un nanocristal d'oxyde de fer, d'oxyde de manganèse ou de fer-platine FePt),
- imagerie optique ou imagerie aux rayons X (l'agent de diagnostic pouvant être un fluorophore lipophile ou un agent de contraste, par exemple une molécule iodée telle que l'iopamidol, l'amidotrizoate, ou des nanoparticules d'or).

De préférence, l'agent de diagnostic est un fluorophore lipophile permettant de réaliser de l'imagerie optique.

La nature du ou des fluorophores lipophiles utilisables n'est pas critique à partir du moment où ils sont compatibles avec l'imagerie in vivo (c'est à dire qu'ils sont biocompatibles et non toxiques). De préférence, les fluorophores utilisés comme agent de diagnostic absorbent et émettent dans le visible et le proche infrarouge, en particulier dans le proche infrarouge. En effet, pour que la lumière d'excitation et la lumière émise par le fluorophore puissent mieux traverser les tissus, il convient d'utiliser des fluorophores absorbant et émettant dans le proche infrarouge, c'est-à-dire à une longueur d'onde comprise entre 640 et 900 nm.

A titre de fluorophore lipophile on peut par exemple citer les composés décrits dans le chapitre 13 ("Probes for Lipids and Membranes") du catalogue InVitrogen. De façon plus précise, on peut notamment citer, à titre de fluorophore, le vert d'indocyanine (ICG), les analogues d'acides gras et les phospholipides fonctionnalisés par un groupement fluorescent tels que les produits fluorescents vendus sous les dénominations commerciales Bodipy (R) tels que le Bodipy (R) 665/676 (Ex/Em.) ; les dérivés amphiphiles de dialkylcarbocyanines telles que le perchlorate de l,r-dioctadécyl-3,3,3',3'-tétraméthylindodicarbocyanine (DiD), par exemple vendu sous la référence D-307 ; les sondes fluorescentes dérivées de sphingolipides, de stéroïdes ou de lipopolysaccharides tels que les produits vendus sous les dénominations commerciales BODIPY ® TR céramides, BODIPY ® FL C5-lactosylcéramide, BODIPY ® FL C5-ganglioside, BODIPY ® FL cérébrosides ; les dérivés amphiphiles de cyanines (tel que le perchlorate de 1,1'-dioctadecyl-3,3,3',3'-tetramethylindodicarbocyanine (DiD)), de rhodamines, de fluorescéines ou de coumarines tels que l'octadécyl rhodamine B, l'octadécyl ester de fluorescéine et la 4-heptadécyl-7- hydroxycoumarine ; et le diphénylhexatriène (DPH) et ses dérivés ; l'ensemble de ces produits étant vendus par la société Invitrogen.

Selon une forme de réalisation préférée de l'Invention, le fluorophore est le vert d'indocyanine ou le perchlorate de l,r-dioctadécyl-3,3,3',3'-tétraméthylindodicarbocyanine.

L'agent thérapeutique pour le traitement de cancers hormonodépendants ou de cancers des organes de synthèse d'hormones stéroïdiennes est notamment choisi parmi :

- les agonistes de la gonadolibérine (ou GnRH, acronyme de l'anglais « Gonadotropin Releasing Hormone », ou encore LHRH, de l'anglais « Luteinizing Hormone Releasing Hormone »), tel que l'oestrogène ou les progestatifs,
- les antagonistes de l'aromatase, tels que le Formestane, l'Exemestane, l'Aminoglutethimide, l'Anastrozole, le Fadrozole, le Letrozole, l'acétate de Megestrol, l'acétate de Medroxyprogestérone ou le Mifepristone, et
- les anti-oestrogènes (pour le traitement du cancer du sein) tels que le Tamoxifène ou le Fulvestrant ou les anti-androgènes (pour le traitement du cancer de la prostate), tels que l'acétate d'abiratérone ou l'acétate de cyprotérone.

Bien entendu, l'agent thérapeutique peut être formulé directement sous sa forme active ou sous forme d'un prodrug.

Par ailleurs, il est envisagé que plusieurs agents thérapeutiques et/ou de diagnostic puissent être formulés en association dans la nanoémulsion.

On cherche généralement à formuler la nanoémulsion avec une concentration maximale en agent thérapeutique ou de diagnostic, notamment lorsqu'il s'agit d'agents peu solubles, afin de limiter le volume et/ou la durée d'administration au patient. Or il a été constaté que la présence du lipide solubilisant dans la phase huileuse permet d'incorporer une quantité importante de ces agents, mêmes hydrophobes ou amphiphiles.

La formulation selon l'invention contiendra le plus souvent une quantité de 0,001 à 30% en poids, de préférence 0,01 à 20% en poids, et encore préférée 0,1 à 10% en poids d'agent thérapeutique ou de diagnostic.

Avantageusement, les agents sont incorporés dans l'émulsion sous forme de solution, et le solvant est ensuite séparé, par exemple par évaporation. La solution contient l'agent thérapeutique ou de diagnostic en une quantité variable pouvant aller jusqu'à sa limite de solubilité. Le choix du solvant dépend de la solubilité de chaque agent thérapeutique ou de diagnostic. Les solvants employés peuvent être par exemple le méthanol, l'éthanol, le chloroforme, le dichlorométhane, l'hexane, le cyclohexane, le DMSO, le DMF ou encore le toluène. De préférence, il s'agit de solvants volatils, de préférence non toxiques pour l'homme.

Selon l'invention, la phase huileuse de la nanoémulsion comporte au moins un lipide amphiphile et au moins un lipide solubilisant.

Afin de former une nanoémulsion stable, il est généralement nécessaire d'inclure dans la composition au moins un lipide amphiphile à titre de tensioactif. La nature amphiphile du tensioactif assure la stabilisation des gouttelettes d'huile au sein de la phase continue aqueuse.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span® par la société Sigma; les lipides polymérisés ; les lipides conjugués à de courtes chaînes d'oxyde de polyéthylène (PEG) tels que les tensioactifs non-ioniques vendus sous les dénominations commerciales Tween® par la société ICI Americas, Inc. et Triton® par la société Union Carbide Corp.; les esters de sucre tels que les mono- et di-laurate, mono- et di-palmitate, mono- et distéarate de saccharose; lesdits tensioactifs pouvant être utilisés seuls ou en mélanges.

Les phospholipides sont les lipides amphiphiles préférés.

La lécithine est le lipide amphiphile particulièrement préféré.

Selon un mode de réalisation particulier, tout ou partie du lipide amphiphile peut posséder une fonction réactive, telle qu'un groupe maléimide, thiol, amine, ester, oxyamine ou aldéhyde. La présence de fonctions réactives permet le greffage de composés fonctionnels au niveau de l'interface. Le lipide amphiphile réactif est incorporé dans la couche formée à l'interface stabilisant la phase dispersée, où il est susceptible de se coupler par exemple avec un composé réactif présent dans la phase aqueuse.

Généralement, la phase huileuse comportera de 0,01 à 99% en poids, de préférence de 5 à 75% en poids, en particulier de 10 à 50% et tout particulièrement de 10 à 30% en poids de lipide amphiphile.

La quantité de lipide amphiphile contribue avantageusement à contrôler la taille de la phase dispersée de la nanoémulsion obtenue.

L'émulsion selon l'invention comprend par ailleurs un lipide solubilisant. Ce composé a pour mission principale de solubiliser le lipide amphiphile, peu soluble, dans la phase huileuse de la nanoémulsion.

Le lipide solubilisant est un lipide présentant une affinité avec le lipide amphiphile suffisante pour permettre sa solubilisation. De préférence, le lipide solubilisant est solide à température ambiante.

Dans le cas où le lipide amphiphile est un phospholipide, il peut s'agir notamment de dérivés du glycérol, et en particulier de glycérides obtenues par estérification de glycérol avec des acides gras.

Le lipide solubilisant utilisé est avantageusement choisi en fonction du lipide amphiphile utilisé. Il présentera généralement une structure chimique proche, afin d'assurer la solubilisation recherchée. Il peut s'agir d'une huile ou d'une cire. De préférence, le lipide solubilisant est solide à température ambiante (20 °C), mais liquide à la température du corps (37 °C).

Les lipides solubilisants préférés, en particulier pour les phospholipides, sont les glycérides d'acides gras, notamment d'acides gras saturés, et en particulier d'acides gras saturés comportant 8 à 18 atomes de carbone, encore préféré 12 à 18 atomes de carbone. Avantageusement, il s'agit d'un mélange de différents glycérides.

De préférence, il s'agit de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, il s'agit de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Particulièrement préférés sont les mélanges de glycérides semi-synthétiques solides à température ambiante vendus sous la dénomination commerciale Suppocire®NC par la société Gattefossé et approuvé pour l'injection chez l'homme. Les Suppocire® de type N sont obtenues par estérification directe d'acides gras et de glycérol. Il s'agit de glycérides hémi-synthétiques d'acides gras saturés de C8 à C18, dont la composition qualiquantitative est indiquée dans le tableau ci-dessous.

Les lipides solubilisants précités permettent d'obtenir une formulation sous forme de nanoémulsion avantageusement stable. Sans vouloir être lié à une théorie particulière, il est supposé que les lipides solubilisants précités permettent d'obtenir des gouttelettes dans la nanoémulsion présentant un coeur amorphe. Le coeur ainsi obtenu présente une viscosité interne élevée sans pour autant présenter de cristallinité. Or, la cristallisation est néfaste pour la stabilité de la nanoémulsion car elle conduit généralement à une agrégation des gouttelettes et/ou à une expulsion des molécules encapsulées à l'extérieur des gouttelettes. Ces propriétés physiques favorisent la stabilité physique de la nanoémulsion

et la stabilité de l'encapsulation au cours du temps de l'agent thérapeutique ou de diagnostic.

La quantité de lipide solubilisant peut varier largement en fonction de la nature et de la quantité de lipide amphiphile présent dans la phase huileuse. Généralement, la phase huileuse comportera de 1 à 99% en poids, de préférence de 5 à 80% en poids et tout particulièrement de 40 à 75% en poids de lipide solubilisant.

Composition en acides gras du Suppocire® NC de Gattefossé

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

La phase huileuse peut comporter par ailleurs une ou plusieurs autres huiles.

Les huiles utilisées présentent de préférence une balance hydrophile-lipophile (HLB) inférieure à 8 et encore plus préférentiellement comprise entre 3 et 6. Avantageusement, les huiles sont utilisées sans modification chimique ou physique préalablement à la formation de l'émulsion.

Selon les applications envisagées, les huiles peuvent être choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de pépin de raisins et de tournesol ; les huiles synthétiques parmi lesquelles figurent notamment les triglycérides, di glycérides et les mono glycérides. Ces huiles peuvent être de premières expressions, raffinées ou inter-estérifiées.

Les huiles préférées sont l'huile de soja et l'huile de lin.

Généralement, si présente, l'huile sera contenue dans la phase huileuse dans une proportion allant de 1 à 80% en poids, de préférence entre 5 et 50 % en poids et tout particulièrement 10 à 30% en poids.

La phase huileuse peut contenir en outre d'autres additifs tels que des colorants, stabilisants, conservateurs ou d'autres principes actifs, en quantité appropriée.

La phase huileuse pour la phase dispersée de l'émulsion peut être préparée par simple mélange des constituants, si nécessaire en chauffant jusqu'à fusion de l'ensemble des constituants.

La phase aqueuse de la nanoémulsion selon l'invention est de préférence constituée d'eau et/ou d'un tampon tel qu'un tampon phosphate comme par exemple du PBS ("Phosphate Buffer Saline") ou d'une solution saline, notamment de chlorure de sodium.

La proportion de phase huileuse et de phase aqueuse est très variable. Cependant, le plus souvent, les nanoémulsions seront préparées avec 1 à 50%, de préférence 5 à 40% et tout particulièrement 10 à 30% en poids de phase huileuse et 50 à 99%, de préférence 60 à 95% et tout particulièrement 70 à 90 % en poids de phase aqueuse.

En outre, la phase aqueuse comporte éventuellement d'autres ingrédients, dont un co-tensioactif. Le co-tensioactif permet de stabiliser la nanoémulsion.

Le co-tensioactif peut par ailleurs présenter d'autres effets dans l'application envisagée de la nanoémulsion.

Les co-tensioactifs utilisables dans les émulsions conformes à la présente invention sont de préférence des tensioactifs hydrosolubles. Ainsi, le co-tensioactif se situe à la fois dans la phase aqueuse continue et dans la phase dispersée. En effet, la partie hydrophobe du co-tensioactif s'insère dans les gouttelettes de la phase dispersée, alors que les chaînes polyalcoxylées sont dans la phase aqueuse continue. Dans la présente demande, les pourcentages massiques de phase dispersée décrits sont calculés en considérant que le co-tensioactif appartient à la phase dispersée.

Les tensioactifs hydrosolubles sont de préférence alkoxylés (ils comprennent au moins une chaîne composée de motifs d'oxyde d'alkylène) et comportent de préférence au moins une chaîne composée de motifs d'oxyde d'éthylène (PEO ou PEG) ou d'oxyde d'éthylène et d'oxyde de propylène. De préférence, le nombre de motifs dans la chaîne varie entre 2 et 500.

A titre d'exemple de co-tensioactifs, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij® (par exemple Brij® 35, 58, 78 ou 98) par la société ICI Americas Inc., les esters d'acide gras et de polyéthylèneglycol tels que les produits vendus sous les dénominations commerciales Myrj® par la société ICI Americas Inc. (par exemple Myrj® 45, 52, 53 ou 59) et les copolymères blocs d'oxyde d'éthylène et

d'oxyde de propylène tels que les produits vendus sous les dénominations commerciales Pluronic® par la société BASF AG (par exemple Pluronic® F68, F127, L64, L61, 10R4, 17R2, 17R4, 25R2 ou 25R4) ou les produits vendus sous la dénomination commerciale Synperonic® par la société Unichema Chemie BV (par exemple Synperonic® PE/F68, PE/L61 ou PE/L64).

La phase aqueuse comporte de 0,01 à 50% en poids, de préférence de 1 à 30% en poids et tout particulièrement de 4 à 20% en poids de co-tensioactif.

Selon un mode de réalisation préféré, la phase dispersée de la nanoémulsion est greffée en surface avec des molécules d'intérêts tels que des ligands biologiques. Un tel greffage permet une reconnaissance spécifique de certaines cellules ou de certains organes. De préférence, le greffage en surface est réalisé par couplage des molécules d'intérêts ou de leurs précurseurs avec un composé amphiphile, notamment avec le co-tensioactif. La nanoémulsion comprend alors un co-tensioactif greffé. Dans ce cas, le composé amphiphile joue le rôle d'un espaceur permettant d'accommoder les molécules d'intérêt en surface.

Les molécules d'intérêt peuvent être par exemple :

- des ligands biologiques de ciblage tels que des anticorps, peptides, saccharides, aptamères, oligonucléotides ou des composés comme l'acide folique ;
- un agent de furtivité : une entité ajoutée afin de conférer à la nanoémulsion une furtivité vis-à-vis du système immunitaire, d'augmenter son temps de circulation dans l'organisme, et de ralentir son élimination.

Par exemple, lorsque le ligand biologique est un peptide comprenant une ou plusieurs cystéine, le greffage à la chaîne oxyde d'alkylène du tensioactif peut être assuré par couplage thiol maléimide.

Dans un mode de réalisation, l'agent de diagnostic ou thérapeutique est greffé au co-tensioactif. Le greffage est typiquement assuré par une liaison covalente entre le co-tensioactif et l'agent de diagnostic ou thérapeutique. Ce mode de réalisation permet d'imposer que l'agent de diagnostic ou thérapeutique se situe à la surface des gouttelettes de la nanoémulsion, à l'interface entre la phase continue et la phase dispersée, ce qui est notamment avantageux lorsque l'agent de diagnostic a un temps de ½ vie courte, par exemple pour des agents de diagnostic comprenant du $^{18}F$ ayant un temps de ½ vie de 118 minutes.

Dans un mode de réalisation, le co-tensioactif de la nanoémulsion est un mélange de co-tensioactifs comprenant un co-tensioactif greffé par un agent de diagnostic ou thérapeutique, un co-tensioactif greffé par une molécule d'intérêt et éventuellement un co-tensioactif non greffé.

Selon un mode de réalisation, la phase aqueuse continue comporte également un agent épaississant tel qu'un glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

La phase aqueuse pour la phase continue de l'émulsion peut être préparée par simple mélange des différents constituants avec le milieu aqueux choisi.

La formulation selon l'invention est avantageusement stable : elle peut être stockée plus de trois mois sans qu'aucune dégradation ne soit observée.

Par ailleurs, le potentiel zêta de la phase dispersée est inférieur à 20 mV en valeur absolue, c'est-à-dire compris entre -20 et 20 mV. Le potentiel zéta élevé en valeur absolue des gouttelettes a pour effet l'accumulation dans le corps, notamment dans le foie, la rate, les poumons, en plus des reins. Or, en particulier pour les applications en imagerie, on recherche à l'inverse une formulation furtive, qui présente une longue durée de vie plasmatique après injection intraveineuse.

**[Procédé de préparation]**

La nanoémulsion telle que décrite peut être préparée aisément par dispersion de quantités appropriées de phase huileuse et de phase aqueuse sous l'effet d'un cisaillement.

Dans le cadre du procédé selon l'invention, on mélange d'abord les différents constituants huileux, le composé de formule (I) et éventuellement l'agent thérapeutique ou de diagnostic pour préparer un pré-mélange huileux pour la phase dispersée de l'émulsion. Le procédé de préparation comprend typiquement les étapes suivantes :

(i) préparer la phase huileuse comprenant au moins un lipide solubilisant, au moins un lipide amphiphile, éventuellement au moins un agent thérapeutique ou de diagnostic et au moins un composé de formule (I) ;

(ii) préparer une phase aqueuse comprenant un co-tensioactif polyalkoxylé ;
(iii) disperser la phase huileuse dans la phase aqueuse sous l'action d'un cisaillement suffisant pour former une nano-émulsion; et
(iv) récupérer la nano-émulsion ainsi formée.

Le mélange peut éventuellement être facilité par mise en solution d'un des constituants ou du mélange complet dans un solvant organique approprié. Le solvant organique est ensuite évaporé, pour obtenir un pré-mélange huileux homogène pour la phase dispersée.
Par ailleurs, il est préféré de réaliser le pré-mélange (étape (i)) à une température à laquelle l'ensemble des ingrédients est liquide.
Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion.
L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une nanoémulsion, le plus souvent quelques minutes.
On obtient alors une nanoémulsion homogène dans laquelle le diamètre moyen des gouttelettes d'huile est supérieur à 10 nm et inférieur à 200 nm, de préférence entre 20 et 50 nm.
De préférence, le potentiel zêta est inférieur à 20 mV en valeur absolue, c'est-à-dire compris entre -20 mV et 20 mV.
Avant conditionnement, l'émulsion peut être diluée et/ou stérilisée, par exemple par filtration ou dialyse. Cette étape permet d'éliminer les éventuels agrégats qui pourraient s'être formés au cours de la préparation de l'émulsion.
L'émulsion ainsi obtenue est prête à l'emploi, le cas échéant après dilution.
Dans les modes de réalisation dans lesquels la nanoémulsion comprend un co-tensioactif greffé, le procédé de préparation comprend typiquement les étapes suivantes :

(i) préparer la phase huileuse comprenant au moins un lipide solubilisant, au moins un lipide amphiphile, éventuellement au moins un agent thérapeutique ou de diagnostic et au moins un composé de formule (I) ;
(ii) préparer une phase aqueuse comprenant un co-tensioactif polyalkoxylé et une fonction susceptible de réagir avec le composé à greffer (molécules d'intérêt ou agent de diagnostique ou thérapeutique);
(iii) disperser la phase huileuse dans la phase aqueuse sous l'action d'un cisaillement suffisant pour former une nano-émulsion;
(iv) ajouter à la nanoémulsion du composé à greffer dans des conditions permettant le greffage entre la fonction susceptible de réagir avec le composé à greffer du co-tensioactif et le composé à greffer,
(v) récupérer la nano-émulsion ainsi formée.

Le greffage est donc typiquement effectué après la formation de la nanoémulsion, ce qui peut être préconisé lorsque les réactions chimiques employées sont compatibles avec la stabilité colloïdale des émulsions, notamment en termes de pH. De préférence, le pH lors de la réaction de greffage est compris entre 5 et 11.
Dans un autre mode de réalisation, le procédé de préparation comprend les étapes suivantes :

(i) fournir un co-tensioactif polyalkoxylé greffé par le composé à greffer (molécules d'intérêt ou agent de diagnostique ou thérapeutique),
(ii) préparer la phase huileuse comprenant au moins un lipide solubilisant, au moins un lipide amphiphile, éventuellement au moins un agent thérapeutique ou de diagnostic et au moins un composé de formule (I) ;
(iii) préparer une phase aqueuse comprenant le co-tensioactif polyalkoxylé greffé par le composé à greffer;
(iv) disperser la phase huileuse dans la phase aqueuse sous l'action d'un cisaillement suffisant pour former une nano-émulsion;
(v) récupérer la nano-émulsion ainsi formée.

Le co-tensioactif polyalkoxylé greffé par le composé à greffer étant généralement obtenu par mise en contact du composé à greffer avec un co-tensioactif comprenant une fonction susceptible de réagir avec le composé à greffer, dans des conditions permettant le greffage entre la fonction susceptible de réagir avec le composé à greffer du co-tensioactif et le composé à greffer.

**[Utilisation]**

Lorsqu'elle est administrée, la formulation selon l'invention cible préférentiellement les cancers hormonodépendants, notamment les cancers du sein ou de la prostate, et les cancers des organes de synthèse d'hormones stéroïdiennes, notamment des ovaires et des surrénales. La spécificité d'un cancer hormonodépendant est qu'il synthétise ses propres hormones stéroïdiennes au sein même de la tumeur.

Sans vouloir être liés à des théories particulières, le ciblage préférentiel des tumeurs (quel que soit leur type) pourrait s'expliquer par l'effet de perméabilité et de rétention (Enhanced Permeability and Rétention (EPR) en anglais) dû à la taille des gouttelettes de la phase dispersée. L'effet EPR est la propriété qu'ont des molécules de certaines tailles (ici les gouttelettes de l'émulsion selon l'invention) à s'accumuler préférentiellement dans le tissu tumoral par rapport au tissu normal. En effet, le tissu tumoral possède une disrégularité au niveau des vaisseaux qui l'alimente permettant aux molécules de certaines tailles (typiquement les liposomes, les nanoparticules ou les médicaments, ici les gouttelettes de la nanoémulsion) de s'accumuler préférentiellement dans le tissu tumoral par rapport au tissu normal. En effet, les cellules tumorales ont une croissance rapide et doivent former au plus vite de nouveaux vaisseaux qui leur permettront un apport en oxygène et en nutriments nécessaires à leur croissance. Due à une synthèse rapide, ces nouveaux vaisseaux vont avoir une forme anormale et une architecture irrégulière. Les cellules n'étant plus alignées, elles laissent apparaître des fenestrations par lesquels les nanoparticules vont pouvoir se faufiler et rentrer au sein de la tumeur. De plus, le tissu tumoral a un faible drainage lymphatique, ce qui va favoriser la rétention des nanoparticules au sein du tissu [Maeda H, Wu J, Sawa T, Matsumura Y, Hori K, J Control Release, 65, 271-284, 2000.].

De plus, il semble que la formulation selon l'invention a une forte affinité pour les zones de synthèse d'hormones stéroïdiennes, et donc les cancers hormonodépendants ou les cancers des organes de synthèse d'hormones stéroïdiennes, pour lesquels la survie, la croissance et la prolifération cellulaire sont sensibles à la présence d'hormones stéroïdiennes : préférentiellement les oestrogènes pour le cancer du sein et préférentiellement les androgènes pour le cancer de la prostate. Tout d'abord, les cellules en général comprennent de nombreux lipides. Les cellules cancéreuses utiliseraient donc les lipides amphiphile et solubilisant de la formulation selon l'invention. De plus, le composé de formule (I) de la formulation selon l'invention a une structure proche des précurseurs utilisés par le corps pour synthétiser les hormones stéroïdiennes. En effet, le composé de formule (I) comprend un groupe A, dont le squelette est stéroïdique. Ainsi, la formulation selon l'invention fournit aux cellules cancéreuses de ces cancers des précurseurs nécessaires à leur prolifération. Les gouttelettes de la formulation selon l'invention seraient attirées préférentiellement par la zone de synthèse d'hormones stéroïdiennes de la tumeur.

Cette théorie pourrait laisser supposer qu'une formulation comprenant n'importe quel composé comportant un squelette stéroïdien, tel que le cholestérol, à la place du composé de formule (I) permettrait de cibler spécifiquement les organes stéroïdiens. Ce n'est pas le cas. La structure particulière des composés de formule (I), qui comprennent un groupe stéroïdique ou stérolique et une chaîne carbonée est importante. En effet, une formulation dans laquelle le composé de formule (I) est remplacé par du cholestérol ne présente pas les mêmes propriétés de stabilité et de ciblage des cancers hormonodépendants ou des cancers des organes de synthèse d'hormones stéroïdiennes, que celles d'une formulation selon la présente invention, comme mis en avant dans l'exemple 3 ci-dessous.

Comme la formulation selon l'invention cible spécifiquement les cancers hormonodépendants, tels que les cancers du sein ou de la prostate, ou les cancers des organes de synthèse d'hormones stéroïdiennes, notamment des ovaires et des surrénales, elle peut être utilisée :

- pour les traiter.
  L'agent thérapeutique pour le traitement de cancers hormonodépendants ou des cancers des organes de synthèse d'hormones stéroïdiennes qu'elle comprend pourra être libéré spécifiquement au niveau du cancer hormonodépendant ou du cancer des organes de synthèse d'hormones stéroïdiennes.
- pour les diagnostiquer.
  La formulation selon l'invention comprenant un agent de diagnostic peut par exemple être utilisée en imagerie peropératoire pour aider le chirurgien lors de la résection de la tumeur, car elle permet de distinguer les zones cancéreuses, les éventuelles métastases ou les ganglions sentinelles.

La formulation selon l'invention peut être utilisée telle qu'elle, ou adaptée à l'application visée, par exemple par dilution, pour l'administration du ou des agents thérapeutiques ou de diagnostic, chez l'homme ou chez l'animal.

Du fait qu'elle peut être préparée exclusivement à partir de constituants approuvés pour l'homme, elle est particulièrement intéressante pour une administration par voie parentérale. Cependant, il est également possible d'envisager une administration par d'autres voies, notamment par voie orale, par voie topique, ou par voie vaginale.

La formulation décrite permet ainsi l'accès à un moyen simple pour l'administration d'agents thérapeutiques nécessaires pour le traitement de cancers hormonodépendants ou de cancers des organes de synthèse d'hormones stéroïdiennes, par voie de chimiothérapie ou photothérapie notamment.

L'invention sera décrite plus en détail au moyen des exemples en annexe.

## EXEMPLES

### EXEMPLE 1 : Préparation de formulations selon l'invention

Le procédé de préparation suivant a été suivi :

(i) Préparation de la phase huileuse :

L'huile de soja, la suppocire NC et la lécithine ont été pesées puis mélangées avec du dichlorométhane avant d'être chauffés à 60°C afin d'obtenir une solution visqueuse homogène. Le dichlorométhane permet de favoriser la solubilisation. Le dichlorométhane est ensuite évaporé sous vide. Le(s) composé(s) de formule (I) a(ont) alors été ajouté(s) dans la phase huileuse obtenue.

(ii) Dopage de la phase huileuse :

Une solution de l'agent de diagnostic (fluorophore) dans l'éthanol a été incorporée à la phase huileuse puis l'éthanol est évaporé sous flux d'azote pendant 5 min. Le mélange a été maintenu visqueux via un bain marie à 40°C / 50°C.

(iii) Préparation de la phase aqueuse :

Pendant la phase d'évaporation de l'éthanol, la phase aqueuse a été préparée. Dans un eppendorf de 5ml, le cotensioactif, le glycérol et la solution aqueuse de PBS ont été mélangés puis dissous dans un bain à 75°C.

(iv) Mélange des deux phases :

La phase huileuse était à environ 40 °C (sous forme visqueuse) et la phase aqueuse à environ 70 °C (en sortie du bain). La phase aqueuse a été versée dans la phase huileuse.

(v) Emulsification :

Le flacon contenant les deux phases a été fixé dans l'enceinte de sonication. La base du flacon (environ 1cm) a été positionnée dans un bain d'eau à 15 °C. La sonotrode conique est plongée dans le flacon, celle-ci étant placée à mi hauteur (sur environ 1 cm dans la solution) pour une meilleure homogénéité de fabrication de la nanoémulsion. La sonication a alors été effectuée avec un sonicateur AV505 (Sonics, Newtown USA). La durée effective de sonication était de 10 min.

(vi) Purification :

La purification a été effectuée par dialyse dans un grand volume de PBS 1X pendant la nuit. Le glycérol est éliminé lors de cette étape.
L'échantillon de nanoémulsion purifié a été prélevé et le volume ajusté par ajout de PBS 1X stérile au volume souhaité (13ml). L'ensemble de l'échantillon a été stérilisé par filtration sur filtre 0,22μm.

Exemple 1.1.: Formulation triplement marquée comprenant deux composés de formule (I) (oléate de cholestéryle marqué et hexadécyléther de cholestéryle marqué) et un fluorophore comme agent de diagnostic (DiD)

Une formulation de composition suivante a été préparée :

Tableau 1 : Composition de la formulation de l'exemple 1.1.

| | | Matière première | Quantité (lot 2ml) | % en masse de la formulation totale | | | % en masse de la phase dispersée (le co-tensioactif appartenant à la phase dispersée) |
|---|---|---|---|---|---|---|---|
| phase huileuse | lipide amphiphile | Lipoid | 17 mg | 0,85 | soit 5,4% | soit premix huileux = 5,50% | 8,42% |
| | lipide solubilisant | Supoccire | 68 mg | 3,4 | | | 33,66% |
| | huile | Huile de soja | 23 mg | 1,15 | | | 11,39% |
| | composé de formule (I) | [3H]CHE | 348 µCi | 0,045 | soit 0,10% | | 0,45% |
| | composé de formule (I) | [14C]CO | 88 µCi | 0,015 | | | 0,15% |
| | agent de diagnostic | DiD | 400µM | 0,04 | | | 0,40% |
| phase aqueuse | co-tensioactif | PEG | 92 mg | 4,6 | - | | 45,53% |
| | solution aqueuse | PBS 1X | 1800 µL | 90 | - | | - |
| | | Phase dispersée | 202,22mg soit 100,11 mg/ml (arrondi à 100 mg/ml) | 10,1 (arrondi à 10) | - | | 100% |

Le DiD est un fluorophore qui peut être détecté par fluorescence.

Les composés [3H]CHE et [14C]CO sont respectivement hexadécyléther de cholestéryle marqué et l'oléate de cholestéryle marqué et ont respectivement les formules suivantes :

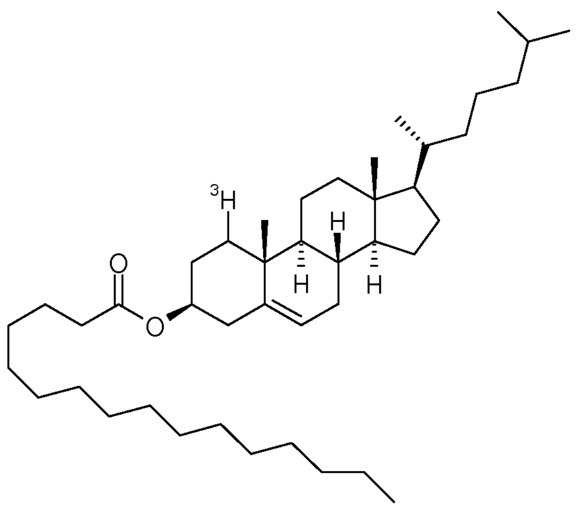

[3H]CHE

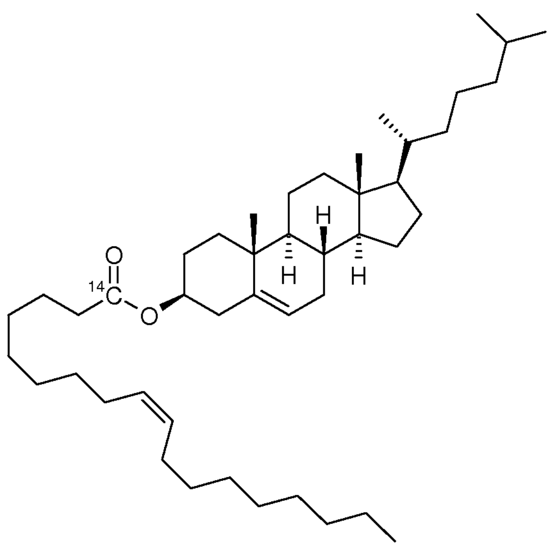

[14C]CO

Ces composés de formule (I) étant marqués, ils peuvent être localisés par mesure de la radioactivité.
La localisation de la nanoémulsion de l'exemple 1.1. peut donc être suivie par fluorescence ou radioactivité.

Exemple 1.2.: Formulation comprenant du stéarate de cholestéryle comme composé de

formule (I) et un fluorophore comme agent de diagnostic (DiD)

Des formulations ont été préparées avec les mêmes compositions que celle du tableau 1, sauf que le [3H]CHE et le [14C]CO sont remplacés par :

- 0,6 %wt par rapport au total de la phase dispersée (en considérant que le co-tensioactif appartient à la phase dispersée) de stéarate de cholestéryle (exemple 1.2.1), ou
- 2 %wt par rapport au total de la phase dispersée (en considérant que le co-tensioactif appartient à la phase dispersée)de stéarate de cholestéryle (exemple 1.2.2).

Le stéarate de cholestéryle est nommé CHST ci-après.
Dans les exemples qui suivent, la radioactivité a été comptée avec le Compteur TRiCarb (Ref : Packard, Liquid Scintillation Analysers ; Models 2200CA).
La Fluobeam 700 (société Fluoptics) est l'appareil avec lequel l'ensemble des organes issus de la biodistribution ont été analysés en fluorescence ainsi que celui avec lequel les cinétiques de biodistribution des nanoémulsions ont été établies in vivo. Ce système d'imagerie permet de visualiser la fluorescence dans les bandes spectrales de 700 à 850 nm pour une excitation de 650 nm. L'appareil est composé d'un boitier électrique renfermant le laser de classe 3B, son alimentation et une tête optique contenant la caméra CDD.
Dans tous les tableaux ci-dessous, MEAN et STD signifient respectivement moyenne et écart-type des mesures.

**EXEMPLE 2 : Résultat in vivo des biodistributions des formulations selon l'invention chez la souris femelle et mâle FVB**

Les nanoémulsions de l'exemple 1 ont été injectées à des souris femelle et mâle FVB.
Pour comparaison, un mélange des trois traceurs ([3H]CHE, [14C]CO et DiD) (non formulés sous forme de nanoémulsion) a été injecté pour observer la distribution des traceurs en l'absence de gouttelettes servant de véhicule de distribution.

Exemple 2.1.: Résultat de biodistribution de la formulation de l'exemple 1.1.

• Exemple comparatif : traceurs libres non encapsulés

Les tableaux 2 à 5 fournissent les résultats de biodistribution de chaque traceur ([3H]CHE, [14C]CO et DiD) exprimés en pourcentage de dose injectée (pour ([3H]CHE et [14C]CO) par gramme de tissu ou en intensité de fluorescence (exprimée en nombre de photons par pixel et ramenée à 100 ms d'intégration du signal, et ce dans tous les tableaux de la présente demande comprenant des valeurs d'intensité de fluorescence) (pour DiD) selon l'organe de la souris, 2 heures (tableau 2), 6 heures (tableau 3), 16 heures (tableau 4) et 24 heures (tableau 5) après injection à une souris femelle FVB d'un mélange des trois traceurs ([3H]CHE, [14C]CO et DiD) non formulés sous forme de nanoémulsion (données comparatives).

Tableau 2

| | 2h | | | | | |
|---|---|---|---|---|---|---|
| | [3H]CHE | | [14C]CO | | DiD | |
| | MEAN | STD | MEAN | STD | MEAN | STD |
| Foie | 1,76 | 0,80 | 4,79 | 1,51 | 2477,20 | 949,88 |
| Intestin | 0,03 | 0,04 | 0,76 | 0,28 | 451,58 | 98,27 |
| Poumon | 29,10 | 14,16 | 72,86 | 30,69 | 848,08 | 299,20 |
| Rate | 1,55 | 0,40 | 3,65 | 0,61 | 1162,07 | 528,53 |
| Rein | 0,09 | 0,05 | 0,89 | 0,41 | 862,35 | 55,97 |
| Gras | 0,02 | 0,02 | 0,69 | 0,49 | 280,30 | 82,36 |

(suite)

| | 2h | | | | | |
|---|---|---|---|---|---|---|
| | [3H]CHE | | [14C]CO | | DiD | |
| | MEAN | STD | MEAN | STD | MEAN | STD |
| Ovaire | 0,04 | 0,03 | 1,57 | 0,78 | 954,70 | 149,20 |
| Surrénales | 0,17 | 0,16 | 3,25 | 1,07 | 730,45 | 392,63 |
| Uterus | 0,02 | 0,01 | 0,49 | 0,05 | 694,28 | 549,88 |
| Cerveau | 0,00 | 0,00 | 0,23 | 0,08 | 439,56 | 61,39 |
| Coeur | 0,08 | 0,08 | 0,59 | 0,09 | 487,37 | 101,42 |
| Muscle | 0,04 | 0,00 | 0,38 | 0,09 | 211,84 | 73,35 |
| Pancreas | 0,05 | 0,04 | 0,47 | 0,10 | 466,58 | 82,71 |
| Salivaire | 0,01 | 0,02 | 0,74 | 0,38 | 399,58 | 178,76 |

Tableau 3

| | 6h | | | | | |
|---|---|---|---|---|---|---|
| | [3H]CHE | | [14C]CO | | DiD | |
| | MEAN | STD | MEAN | STD | MEAN | STD |
| Foie | 3,23 | 1,88 | 5,53 | 3,71 | 2293,21 | 901,09 |
| Intestin | 0,15 | 0,11 | 1,21 | 0,37 | 363,09 | 127,53 |
| Poumon | 83,99 | 53,84 | 142,66 | 81,34 | 433,52 | 111,50 |
| Rate | 3,35 | 1,64 | 3,53 | 2,11 | 3224,12 | 645,77 |
| Rein | 0,41 | 0,17 | 1,36 | 0,49 | 573,78 | 97,58 |
| Gras | 0,05 | 0,10 | 1,05 | 0,37 | 234,61 | 88,10 |
| Ovaire | 0,07 | 0,08 | 2,12 | 0,20 | 250,27 | 338,83 |
| Surrénales | 0,36 | 0,23 | 3,56 | 0,36 | 208,14 | 196,51 |
| Uterus | 0,04 | 0,01 | 0,46 | 0,09 | 506,03 | 78,54 |
| Cerveau | 0,02 | 0,02 | 0,26 | 0,04 | 116,17 | 78,03 |
| Coeur | 0,30 | 0,18 | 1,21 | 0,59 | 346,63 | 60,44 |
| Muscle | 0,03 | 0,03 | 0,53 | 0,16 | 108,34 | 29,11 |
| Pancreas | 0,09 | 0,04 | 0,76 | 0,13 | 191,73 | 50,40 |
| Salivaire | 0,09 | 0,01 | 1,15 | 0,14 | 232,98 | 151,37 |

Tableau 4

| | 16h | | | | | |
|---|---|---|---|---|---|---|
| | [3H]CHE | | [14C]CO | | DiD | |
| | MEAN | STD | MEAN | STD | MEAN | STD |
| Foie | 6,95 | 0,79 | 11,86 | 2,81 | 2954,59 | 420,30 |
| Intestin | 0,13 | 0,05 | 1,09 | 0,33 | 505,69 | 190,99 |
| Poumon | 124,35 | 61,28 | 261,87 | 130,59 | 370,94 | 27,96 |
| Rate | 4,17 | 0,17 | 5,32 | 1,21 | 3403,29 | 953,61 |
| Rein | 0,53 | 0,06 | 1,82 | 0,26 | 382,29 | 194,01 |
| Gras | 0,02 | 0,02 | 1,39 | 0,38 | 295,69 | 118,05 |
| Ovaire | 0,19 | 0,16 | 1,64 | 0,39 | 729,53 | 604,29 |
| Surrénales | 0,47 | 0,22 | 4,24 | 1,01 | 1683,36 | 399,48 |
| Uterus | 0,03 | 0,02 | 1,00 | 0,27 | 665,40 | 284,46 |

(suite)

| | 16h | | | | | |
|---|---|---|---|---|---|---|
| | [3H]CHE | | [14C]CO | | DiD | |
| | MEAN | STD | MEAN | STD | MEAN | STD |
| Cerveau | 0,02 | 0,02 | 0,30 | 0,07 | -24,23 | 53,01 |
| Coeur | 0,35 | 0,17 | 1,87 | 0,13 | 186,72 | 102,04 |
| Muscle | 0,05 | 0,01 | 0,43 | 0,15 | 93,88 | 56,25 |
| Pancreas | 0,04 | 0,04 | 1,00 | 0,07 | 101,87 | 104,38 |
| Salivaire | 0,05 | 0,05 | 1,29 | 0,22 | 271,01 | 172,82 |

Tableau 5

| | 24h | | | | | |
|---|---|---|---|---|---|---|
| | [3H]CHE | | [14C]CO | | DiD | |
| | MEAN | STD | MEAN | STD | MEAN | STD |
| Foie | 1,12 | 0,93 | 3,29 | 3,51 | 2663,45 | 439,54 |
| Intestin | 0,07 | 0,04 | 0,81 | 0,28 | 354,94 | 252,57 |
| Poumon | 6,37 | 6,75 | 29,91 | 34,49 | 300,36 | 72,13 |
| Rate | 10,63 | 15,66 | 2,27 | 1,90 | 3880,70 | 147,54 |
| Rein | 1,12 | 1,69 | 1,02 | 0,43 | 397,90 | 234,64 |
| Gras | 1,42 | 2,36 | 1,64 | 0,39 | 344,99 | 37,37 |
| Ovaire | 0,08 | 0,07 | 1,40 | 0,51 | 989,32 | 137,18 |
| Surrénales | 0,31 | 0,25 | 2,60 | 0,77 | 653,68 | 140,78 |
| Uterus | 0,34 | 0,53 | 0,62 | 0,12 | 636,69 | 101,11 |
| Cerveau | 0,03 | 0,05 | 0,22 | 0,03 | 57,68 | 51,05 |
| Coeur | 0,12 | 0,05 | 0,82 | 0,37 | 285,67 | 92,94 |
| Muscle | 0,11 | 0,01 | 0,79 | 0,27 | 289,03 | 383,41 |
| Pancreas | 0,03 | 0,02 | 0,95 | 0,63 | 158,45 | 65,37 |
| Salivaire | 0,14 | 0,13 | 0,86 | 0,50 | 187,28 | 176,21 |

Les tableaux 2 à 5 montrent une forte accumulation des traceurs radioactifs libres ([3H]CHE et [14C]CO) dans les poumons. Une forte accumulation dans le foie et la rate est observée pour le traceur DiD seul.

• Nanoémulsion selon l'exemple 1.1. injectée à une souris femelle FVB

Les tableaux 6 à 14 fournissent les résultats de biodistribution de chaque traceur ([3H]CHE, [14C]CO et DiD) exprimés en pourcentage de dose injectée par gramme de tissu (pour [3H]CHE et [14C]CO) ou en intensité de fluorescence (pour DiD) selon l'organe de la souris, 15 minutes (tableau 6), 2 heures (tableau 7), 4 heures (tableau 8), 6 heures (tableau 9), 16 heures (tableau 10), 24 heures (tableau 11), 72 heures (tableau 12), 120 heures (tableau 13) et 168 heures (tableau 14), après injection à une souris femelle FVB de la nanoémulsion de l'exemple 1.1. (nanoémulsion selon l'invention) comprenant les trois traceurs ([3H]CHE, [14C]CO et DiD).

Tableau 6

| | 15min | | | | | |
|---|---|---|---|---|---|---|
| | [3H]CHE | | [14C]CO | | DiD | |
| Organes | MEAN | STD | MEAN | STD | MEAN | STD |
| Foie | 8,26 | 1,27 | 8,86 | 1,85 | 979 | 174 |
| Intestin | 1,71 | 0,87 | 1,75 | 0,81 | 300 | 262 |

(suite)

| | 15min | | | | | |
|---|---|---|---|---|---|---|
| | [3H]CHE | | [14C]CO | | DiD | |
| Organes | MEAN | STD | MEAN | STD | MEAN | STD |
| Poumons | 3,91 | 1,65 | 3,92 | 1,81 | 618 | 391 |
| Rate | 3,63 | 0,88 | 3,58 | 0,82 | 727 | 182 |
| Rein | 4,09 | 2,70 | 4,20 | 2,76 | 228 | 125 |
| Gras | 1,10 | 0,54 | 1,15 | 0,51 | 147 | 130 |
| Ovaire | 5,38 | 3,33 | 4,59 | 2,62 | 484 | 616 |
| Surrénales | 10,60 | 3,57 | 10,18 | 2,89 | 457 | 366 |
| Utérus | 0,90 | 0,29 | 0,93 | 0,29 | 178 | 142 |
| Cerveau | 0,476 | 0,047 | 0,499 | 0,052 | 145 | 55 |
| Coeur | 8,303 | 6,540 | 8,676 | 6,814 | 451 | 136 |
| Muscle | 0,675 | 0,677 | 0,744 | 0,824 | 1 | 11 |
| Pancréas | 1,233 | 0,298 | 1,262 | 0,244 | 216 | 145 |
| Salivaires | 1,157 | 0,562 | 1,057 | 0,312 | 59 | 190 |

Tableau 7

| | 2h | | | | | |
|---|---|---|---|---|---|---|
| | [3H]CHE | | [14C]CO | | DiD | |
| Organes | MEAN | STD | MEAN | STD | MEAN | STD |
| Foie | 31,09 | 4,34 | 19,41 | 3,24 | 1421 | 142 |
| Intestin | 2,44 | 0,13 | 2,33 | 0,19 | 637 | 377 |
| Poumons | 4,44 | 1,49 | 4,42 | 1,25 | 727 | 406 |
| Rate | 5,10 | 1,10 | 2,87 | 0,42 | 805 | 304 |
| Rein | 3,83 | 0,29 | 3,91 | 0,23 | 473 | 70 |
| Gras | 0,76 | 0,17 | 3,85 | 1,04 | 247 | 102 |
| Ovaire | 29,28 | 14,72 | 22,72 | 12,69 | 1807 | 661 |
| Surrénales | 21,12 | 4,59 | 18,50 | 3,42 | 1050 | 340 |
| Utérus | 2,23 | 0,11 | 2,21 | 0,20 | 814 | 50 |
| Cerveau | 0,26 | 0,09 | 0,29 | 0,08 | 250 | 120 |
| Coeur | 4,64 | 1,18 | 4,41 | 1,17 | 389 | 157 |
| Muscle | 0,45 | 0,12 | 0,48 | 0,09 | 213 | 28 |
| Pancréas | 1,43 | 0,10 | 1,68 | 0,15 | 399 | 59 |
| Salivaires | 1,42 | 0,70 | 1,74 | 0,56 | 288 | 221 |

Tableau 8

| | 4h | | | | | |
|---|---|---|---|---|---|---|
| | [3H]CHE | | [14C]CO | | DiD | |
| Organes | MEAN | STD | MEAN | STD | MEAN | STD |
| Foie | 37,43 | 5,51 | 13,69 | 1,62 | 1845 | 245 |
| Intestin | 2,46 | 0,56 | 1,80 | 0,39 | 974 | 152 |
| Poumons | 4,02 | 0,34 | 3,09 | 0,19 | 925 | 81 |
| Rate | 5,00 | 0,93 | 1,97 | 0,22 | 955 | 19 |
| Rein | 2,51 | 0,32 | 2,27 | 0,34 | 614 | 108 |

(suite)

| | 4h | | | | | |
|---|---|---|---|---|---|---|
| | [3H]CHE | | [14C]CO | | DiD | |
| Organes | MEAN | STD | MEAN | STD | MEAN | STD |
| Gras | 1,40 | 1,17 | 1,46 | 0,44 | 522 | 311 |
| Ovaire | 39,41 | 11,65 | 25,14 | 6,94 | 2781 | 181 |
| Surrénales | 28,13 | 3,23 | 21,37 | 3,98 | 1754 | 385 |
| Utérus | 1,69 | 1,42 | 1,39 | 1,11 | 712 | 538 |
| Cerveau | 0,19 | 0,02 | 0,23 | 0,04 | 279 | 74 |
| Coeur | 4,94 | 1,05 | 3,75 | 0,49 | 550 | 87 |
| Muscle | 0,41 | 0,11 | 0,38 | 0,09 | 236 | 64 |
| Pancréas | 1,01 | 0,26 | 1,17 | 0,18 | 527 | 138 |
| Salivaires | 1,09 | 0,19 | 1,47 | 0,13 | 516 | 114 |

Tableau 9

| | 16h | | | | | |
|---|---|---|---|---|---|---|
| | [3H]CHE | | [14C]CO | | DiD | |
| Organes | MEAN | STD | MEAN | STD | MEAN | STD |
| Foie | 47,67 | 7,58 | 8,68 | 3,35 | 1661 | 342 |
| Intestin | 4,87 | 0,93 | 2,53 | 0,37 | 1139 | 290 |
| Poumons | 4,63 | 1,97 | 2,80 | 0,94 | 592 | 169 |
| Rate | 7,48 | 0,51 | 1,89 | 0,19 | 978 | 434 |
| Rein | 2,06 | 0,15 | 1,75 | 0,08 | 627 | 79 |
| Gras | 1,36 | 0,86 | 8,06 | 1,53 | 532 | 248 |
| Ovaire | 52,26 | 2,35 | 21,45 | 2,85 | 2915 | 184 |
| Surrénales | 43,16 | 14,73 | 30,64 | 9,04 | 2355 | 328 |
| Utérus | 3,91 | 1,01 | 2,76 | 0,98 | 1759 | 1216 |
| Cerveau | 0,13 | 0,01 | 0,13 | 0,01 | 259 | 42 |
| Coeur | 3,49 | 0,66 | 2,00 | 0,41 | 444 | 123 |
| Muscle | 0,76 | 0,26 | 1,59 | 0,48 | 289 | 43 |
| Pancréas | 1,17 | 0,44 | 1,19 | 0,35 | 564 | 233 |
| Salivaires | 1,27 | 0,79 | 2,02 | 1,05 | 641 | 238 |

Tableau 10

| | 24h | | | | | |
|---|---|---|---|---|---|---|
| | [3H]CHE | | [14C]CO | | DiD | |
| Organes | MEAN | STD | MEAN | STD | MEAN | STD |
| Foie | 48,76 | 7,87 | 5,39 | 1,95 | 1675 | 299 |
| Intestin | 2,78 | 1,00 | 1,22 | 0,20 | 777 | 215 |
| Poumons | 3,21 | 0,55 | 1,30 | 0,29 | 667 | 124 |
| Rate | 7,50 | 2,34 | 1,10 | 0,11 | 774 | 85 |
| Rein | 1,50 | 0,40 | 1,03 | 0,22 | 599 | 47 |
| Gras | 0,88 | 0,43 | 2,28 | 1,08 | 553 | 472 |
| Ovaire | 38,06 | 14,16 | 9,58 | 2,88 | 3173 | 370 |
| Surrénales | 39,68 | 14,88 | 13,05 | 4,09 | 2624 | 460 |

(suite)

| | 24h | | | | | |
|---|---|---|---|---|---|---|
| | [3H]CHE | | [14C]CO | | DiD | |
| Organes | MEAN | STD | MEAN | STD | MEAN | STD |
| Utérus | 1,82 | 0,87 | 0,96 | 0,48 | 971 | 474 |
| Cerveau | 0,09 | 0,01 | 0,11 | 0,01 | 239 | 40 |
| Coeur | 3,53 | 0,62 | 1,31 | 0,25 | 288 | 54 |
| Muscle | 0,46 | 0,17 | 1,01 | 0,53 | 161 | 52 |
| Pancréas | 0,64 | 0,13 | 0,67 | 0,04 | 446 | 113 |
| Salivaires | 1,40 | 0,61 | 1,76 | 0,07 | 411 | 186 |

Tableau 11

| | 48h | | | | | |
|---|---|---|---|---|---|---|
| | [3H]CHE | | [14C]CO | | DiD | |
| Organes | MEAN | STD | MEAN | STD | MEAN | STD |
| Foie | 58,43 | 2,25 | 1,53 | 0,37 | 1868 | 258 |
| Intestin | 4,43 | 0,38 | 1,01 | 0,04 | 481 | 77 |
| Poumons | 3,91 | 0,21 | 1,28 | 0,10 | 212 | 39 |
| Rate | 12,32 | 2,48 | 1,03 | 0,05 | 342 | 71 |
| Rein | 1,63 | 0,19 | 0,77 | 0,08 | 555 | 305 |
| Gras | 1,05 | 0,26 | 3,46 | 2,81 | 208 | 31 |
| Ovaire | 36,42 | 8,45 | 6,46 | 1,12 | 4018 | 668 |
| Surrénales | 42,26 | 1,49 | 10,83 | 0,49 | 1484 | 607 |
| Utérus | 3,75 | 2,06 | 1,24 | 0,51 | 951 | 697 |
| Cerveau | 0,08 | 0,01 | 0,11 | 0,00 | 51 | 47 |
| Coeur | 3,52 | 0,23 | 0,87 | 0,04 | 122 | 72 |
| Muscle | 0,36 | 0,05 | 0,34 | 0,18 | 32 | 39 |
| Pancréas | 1,00 | 0,29 | 0,70 | 0,21 | 202 | 102 |
| Salivaires | 1,09 | 0,16 | 1,20 | 0,61 | 268 | 103 |

Tableau 12

| | 72h | | | | | |
|---|---|---|---|---|---|---|
| | [3H]CHE | | [14C]CO | | DiD | |
| Organes | MEAN | STD | MEAN | STD | MEAN | STD |
| Foie | 39,73 | 8,94 | 0,75 | 0,11 | 1136 | 392 |
| Intestin | 3,58 | 1,96 | 0,62 | 0,23 | 297 | 115 |
| Poumons | 2,95 | 0,54 | 0,88 | 0,18 | 120 | 169 |
| Rate | 7,65 | 3,13 | 0,75 | 0,21 | 330 | 172 |
| Rein | 1,27 | 0,30 | 0,66 | 0,09 | 365 | 129 |
| Gras | 0,85 | 0,14 | 5,01 | 3,51 | 159 | 51 |
| Ovaire | 25,11 | 6,58 | 3,04 | 0,81 | 2397 | 325 |
| Surrénales | 36,92 | 14,04 | 9,36 | 5,06 | 1135 | 586 |
| Utérus | 5,63 | 5,79 | 1,00 | 0,66 | 514 | 552 |
| Cerveau | 0,08 | 0,02 | 0,10 | 0,02 | 43 | 35 |
| Coeur | 2,98 | 0,88 | 0,64 | 0,19 | 113 | 26 |

(suite)

| | 72h | | | | | |
|---|---|---|---|---|---|---|
| | [3H]CHE | | [14C]CO | | DiD | |
| Organes | MEAN | STD | MEAN | STD | MEAN | STD |
| Muscle | 0,22 | 0,04 | 0,28 | 0,05 | 3 | 47 |
| Pancréas | 0,74 | 0,20 | 0,50 | 0,12 | 102 | 38 |
| Salivaires | 1,10 | 0,27 | 1,16 | 0,43 | 237 | 114 |

Tableau 13

| | 120h | | | | | |
|---|---|---|---|---|---|---|
| | [3H]CHE | | [14C]CO | | DiD | |
| Organes | MEAN | STD | MEAN | STD | MEAN | STD |
| Foie | 39,13 | 3,21 | 0,42 | 0,03 | 996 | 280 |
| Intestin | 3,60 | 0,24 | 0,28 | 0,03 | 279 | 24 |
| Poumons | 3,30 | 0,75 | 0,57 | 0,05 | 53 | 55 |
| Rate | 9,69 | 1,34 | 0,45 | 0,04 | 229 | 98 |
| Rein | 1,29 | 0,12 | 0,40 | 0,05 | 383 | 59 |
| Gras | 0,68 | 0,10 | 2,42 | 1,32 | 176 | 65 |
| Ovaire | 16,92 | 11,11 | 1,24 | 0,50 | 2319 | 815 |
| Surrénales | 41,92 | 8,51 | 6,43 | 0,63 | 1688 | 136 |
| Utérus | 4,99 | 3,67 | 0,62 | 0,41 | 563 | 507 |
| Cerveau | 0,08 | 0,00 | 0,08 | 0,00 | 90 | 36 |
| Coeur | 2,59 | 0,54 | 0,37 | 0,03 | 80 | 32 |
| Muscle | 0,27 | 0,02 | 0,30 | 0,05 | 254 | 348 |
| Pancréas | 1,00 | 0,34 | 0,40 | 0,16 | 297 | 49 |
| Salivaires | 2,14 | 0,84 | 1,78 | 1,01 | 120 | 339 |

Tableau 14

| | 168h | | | | | |
|---|---|---|---|---|---|---|
| | [3H]CHE | | [14C]CO | | DiD | |
| Organes | MEAN | STD | MEAN | STD | MEAN | STD |
| Foie | 32,70 | 6,86 | 0,28 | 0,01 | 676,60 | 14,849 |
| Intestin | 2,80 | 0,80 | 0,18 | 0,02 | 214,02 | 23,794 |
| Poumons | 1,70 | 0,14 | 0,25 | 0,06 | 1,35 | 0,120 |
| Rate | 10,71 | 0,49 | 0,28 | 0,00 | 171,93 | 88,862 |
| Rein | 1,44 | 0,19 | 0,28 | 0,03 | 391,45 | 128,679 |
| Gras | 0,99 | 0,14 | 5,02 | 2,61 | 164,55 | 115,690 |
| Ovaire | 20,61 | 4,67 | 0,74 | 0,08 | 1614,45 | 387,463 |
| Surrénales | 29,30 | 3,35 | 2,73 | 0,04 | 660,62 | 704,034 |
| Utérus | 4,86 | 0,63 | 0,37 | 0,03 | 697,92 | 378,217 |
| Cerveau | 0,07 | 0,02 | 0,07 | 0,00 | 40,28 | 1,563 |
| Coeur | 2,19 | 0,44 | 0,22 | 0,03 | 117,29 | 17,600 |
| Muscle | 0,37 | 0,19 | 0,32 | 0,08 | 16,21 | 54,384 |
| Pancréas | 0,79 | 0,12 | 0,23 | 0,00 | 125,11 | 68,066 |
| Salivaires | 2,01 | 0,93 | 1,14 | 0,19 | 216,84 | 196,130 |

La comparaison des valeurs des tableaux 2 à 5 et des tableaux 6 à 14 montre que la biodistribution est différente lorsque des traceurs libres sont injectés ou lorsque la nanoémulsion selon l'exemple 1.1 est injectée. Dans le cas de l'utilisation d'une nanoémulsion selon l'invention, les trois traceurs vont au même endroit au même moment jusqu'à un temps d'environ 12h post injection, ce qui démontre l'effet vecteur (ciblage) des gouttelettes de la nanoémulsion selon l'invention.

Une accumulation des gouttelettes de la nanoémulsion selon l'invention est observée dans le foie (zone de métabolisation des nanogouttelettes lipidiques) ainsi que dans les organes stéroïdiens (ovaires et surrénales), qui sont les organes de synthèse des hormones stéroïdiennes. Ces mêmes zones de synthèse des hormones stéroïdiennes se retrouvent dans les tissus cancéreux des cancers hormonodépendants, tels que par exemple certains cancers du sein ou de la prostate.

Un léger signal de fluorescence est observé dans l'utérus. Il a été observé après dissection et histologie, que suivant la période du cycle de la souris, la morphologie de l'utérus change ainsi que son autofluorescence qui devient plus élevée. Ainsi, ces résultats proviendraient de l'autofluorescence de l'utérus.

Comme une accumulation préférentielle dans les ovaires et les surrénales a été observée, des images de fluorescence ont été réalisées en cryohistologie. Les images de fluorescence à 680 nm confirment l'accumulation des gouttelettes de la nanoémulsion dans ces organes. Les images en histologie des ovaires montrent une accumulation dans le corps jaune (corpus luteum), qui a pour fonction de secréter la progestérone. Les images en histologie des surrénales montrent une accumulation dans la zone corticale, qui assure la sécrétion des stéroïdes.

• Nanoémulsion selon l'exemple 1.1. injectée à une souris mâle FVB

Les tableaux 15 à 21 fournissent les résultats de biodistribution de chaque traceur ([3H]CHE, [14C]CO et DiD) exprimés en pourcentage de dose injectée par gramme de tissu (pour [3H]CHE et [14C]CO) ou en intensité de fluorescence (pour DiD) selon l'organe de la souris, 15 minutes (tableau 6), 2 heures (tableau 7), 4 heures (tableau 8), 6 heures (tableau 9), 16 heures (tableau 10), 24 heures (tableau 11), 72 heures (tableau 12), 120 heures (tableau 13) et 168 heures (tableau 14), après injection à une souris mâle FVB de la nanoémulsion de l'exemple 1.1. comprenant les trois traceurs ([3H]CHE, [14C]CO et DiD).

Tableau 15

| | 2h | | | | | |
|---|---|---|---|---|---|---|
| | [3H]CHE | | [14C]CO | | DiD | |
| | Mean | STD | Mean | STD | Mean | STD |
| Foie | 22,67 | 1,50 | 11,13 | 1,40 | 831,9 | 234,8 |
| Intestin | 2,44 | 0,17 | 2,05 | 0,25 | 392,1 | 78,3 |
| Poumon | 5,08 | 0,80 | 4,45 | 0,90 | 559,9 | 124,5 |
| Rate | 7,15 | 0,49 | 2,97 | 0,36 | 455,7 | 216,8 |
| Rein | 5,29 | 0,90 | 6,10 | 1,35 | 601,1 | 79,7 |
| Gras | 1,14 | 0,19 | 1,62 | 0,44 | 184,0 | 31,0 |
| Testicule | 1,36 | 0,12 | 1,34 | 0,12 | 639,9 | 141,0 |
| Surénale | 10,16 | 2,47 | 8,63 | 3,25 | 779,1 | 21,6 |
| Séminales | 0,79 | 0,28 | 0,80 | 0,28 | 175,3 | 64,6 |
| Cerveau | 0,35 | 0,03 | 0,40 | 0,05 | 186,5 | 47,7 |
| Coeur | 9,84 | 1,09 | 9,28 | 1,36 | 427,6 | 55,3 |
| Muscle | 0,99 | 0,24 | 0,94 | 0,27 | 176,7 | 54,4 |
| Pancréas | 1,64 | 0,15 | 1,72 | 0,09 | 255,1 | 99,4 |
| Salivaires | 1,53 | 0,25 | 1,99 | 0,37 | 310,0 | 97,2 |

Tableau 16

| | 4h | | | | | |
|---|---|---|---|---|---|---|
| | [3H]CHE | | [14C]CO | | DiD | |
| | Mean | STD | Mean | STD | Mean | STD |
| Foie | 17,25 | 5,85 | 5,76 | 1,18 | 813,0 | 71,6 |
| Intestin | 1,98 | 0,68 | 1,49 | 0,50 | 336,2 | 108,5 |
| Poumon | 2,98 | 1,44 | 2,30 | 1,04 | 446,4 | 105,1 |
| Rate | 5,31 | 1,82 | 2,08 | 0,41 | 407,4 | 151,7 |
| Rein | 3,14 | 0,96 | 3,36 | 1,13 | 505,0 | 127,1 |
| Gras | 0,74 | 0,20 | 0,71 | 0,15 | 128,3 | 55,5 |
| Testicule | 1,35 | 0,33 | 1,26 | 0,29 | 470,9 | 283,7 |
| Surénale | 16,96 | 7,65 | 15,01 | 5,05 | 536,1 | 177,9 |
| Séminales | 0,50 | 0,17 | 0,52 | 0,16 | 179,5 | 39,8 |
| Cerveau | 0,23 | 0,07 | 0,24 | 0,06 | 150,6 | 28,3 |
| Coeur | 7,57 | 2,16 | 6,07 | 1,93 | 306,9 | 52,4 |
| Muscle | 0,56 | 0,06 | 0,51 | 0,02 | 152,3 | 56,0 |
| Pancréas | 1,50 | 0,40 | 1,54 | 0,43 | 295,0 | 58,7 |
| Salivaires | 1,26 | 0,20 | 1,70 | 0,18 | 297,8 | 130,0 |

Tableau 17

| | 6h | | | | | |
|---|---|---|---|---|---|---|
| | [3H]CHE | | [14C]CO | | DiD | |
| | Mean | STD | Mean | STD | Mean | STD |
| Foie | 22,609 | 3,289 | 5,926 | 0,706 | 847,4 | 301,5 |
| Intestin | 2,549 | 0,564 | 1,778 | 0,434 | 378,0 | 54,4 |
| Poumon | 3,771 | 0,541 | 2,790 | 0,325 | 426,0 | 181,2 |
| Rate | 6,453 | 1,102 | 2,025 | 0,135 | 488,6 | 129,1 |
| Rein | 3,336 | 0,460 | 3,841 | 0,775 | 427,7 | 68,0 |
| Gras | 1,234 | 0,574 | 1,217 | 0,633 | 221,1 | 69,1 |
| Testicule | 2,000 | 0,632 | 1,839 | 0,642 | 723,1 | 112,0 |
| Surénale | 13,399 | 2,214 | 10,940 | 1,302 | 721,9 | 38,7 |
| Séminales | 0,54 | 0,16 | 0,59 | 0,19 | 261,0 | 32,1 |
| Cerveau | 0,24 | 0,04 | 0,28 | 0,04 | 235,9 | 42,1 |
| Coeur | 8,52 | 0,69 | 6,10 | 0,23 | 253,8 | 75,3 |
| Muscle | 0,77 | 0,10 | 0,58 | 0,04 | 169,3 | 40,2 |
| Pancréas | 1,67 | 0,32 | 1,55 | 0,38 | 347,0 | 90,3 |
| Salivaires | 2,10 | 1,24 | 1,95 | 0,52 | 488,5 | 50,8 |

Tableau 18

| | 16h | | | | | |
|---|---|---|---|---|---|---|
| | [3H]CHE | | [14C]CO | | DiD | |
| | Mean | STD | Mean | STD | MEAN | STD |
| Foie | 40,91 | 1,28 | 3,55 | 0,34 | 1157,2 | 433,7 |
| Intestin | 5,19 | 0,59 | 2,13 | 0,21 | 640,4 | 298,4 |
| Poumon | 5,31 | 0,91 | 2,14 | 0,42 | 423,5 | 94,4 |

(suite)

| | 16h | | | | | |
|---|---|---|---|---|---|---|
| | [3H]CHE | | [14C]CO | | DiD | |
| | Mean | STD | Mean | STD | MEAN | STD |
| Rate | 9,19 | 1,06 | 1,25 | 0,14 | 373,3 | 96,6 |
| Rein | 2,40 | 0,31 | 2,01 | 0,26 | 343,0 | 61,3 |
| Gras | 1,70 | 1,13 | 2,24 | 0,88 | 117,0 | 6,5 |
| Testicule | 2,95 | 0,50 | 2,14 | 0,41 | 525,4 | 119,1 |
| Surénale | 32,89 | 1,11 | 17,48 | 5,45 | 1086,2 | 242,6 |
| Séminales | 0,64 | 0,21 | 0,77 | 0,33 | 163,0 | 128,4 |
| Cerveau | 0,18 | 0,01 | 0,17 | 0,01 | 134,7 | 73,7 |
| Coeur | 8,50 | 0,94 | 3,22 | 0,21 | 281,5 | 85,1 |
| Muscle | 0,97 | 0,25 | 0,72 | 0,20 | 281,9 | 83,9 |
| Pancréas | 1,92 | 0,60 | 1,35 | 0,37 | 332,6 | 131,2 |
| Salivaires | 2,68 | 0,17 | 2,11 | 0,35 | 838,8 | 227,5 |

Tableau 19

| | 24h | | | | | |
|---|---|---|---|---|---|---|
| | [3H]CHE | | [14C]CO | | DiD | |
| | Mean | STD | Mean | STD | MEAN | STD |
| Foie | 30,93 | 7,18 | 2,05 | 0,42 | 1152,7 | 120,1 |
| Intestin | 3,41 | 0,80 | 1,18 | 0,22 | 621,0 | 64,0 |
| Poumon | 2,88 | 0,80 | 1,08 | 0,03 | 406,0 | 28,6 |
| Rate | 6,65 | 1,60 | 0,80 | 0,12 | 638,0 | 197,6 |
| Rein | 1,80 | 0,26 | 1,11 | 0,11 | 323,1 | 10,6 |
| Gras | 0,83 | 0,50 | 0,60 | 0,33 | 142,1 | 54,5 |
| Testicule | 1,71 | 1,29 | 1,08 | 0,66 | 575,9 | 104,8 |
| Surénale | 17,41 | 5,59 | 7,78 | 3,66 | 1105,9 | 126,9 |
| Séminales | 0,35 | 0,28 | 0,30 | 0,24 | 170,6 | 43,2 |
| Cerveau | 0,11 | 0,03 | 0,12 | 0,03 | 109,3 | 16,6 |
| Coeur | 5,76 | 2,10 | 1,72 | 0,71 | 239,7 | 17,6 |
| Muscle | 0,38 | 0,31 | 0,38 | 0,08 | 144,9 | 28,5 |
| Pancréas | 1,31 | 0,12 | 1,14 | 0,57 | 268,9 | 38,7 |
| Salivaires | 1,57 | 0,78 | 1,69 | 1,21 | 501,1 | 155,2 |

Tableau 20

| | 48h | | | | | |
|---|---|---|---|---|---|---|
| | [3H]CHE | | [14C]CO | | DiD | |
| | Mean | STD | Mean | STD | MEAN | STD |
| Foie | 24,53 | 0,76 | 0,70 | 0,13 | 988,2 | 149,5 |
| Intestin | 3,50 | 0,61 | 0,55 | 0,03 | 381,6 | 116,6 |
| Poumon | 3,02 | 0,02 | 0,67 | 0,10 | 346,5 | 115,9 |
| Rate | 11,18 | 3,50 | 0,76 | 0,01 | 656,7 | 132,2 |
| Rein | 1,77 | 0,44 | 0,82 | 0,34 | 168,3 | 34,4 |
| Gras | 0,94 | 0,18 | 0,85 | 0,34 | 185,1 | 62,2 |

(suite)

| | 48h | | | | | |
|---|---|---|---|---|---|---|
| | [3H]CHE | | [14C]CO | | DiD | |
| | Mean | STD | Mean | STD | MEAN | STD |
| Testicule | 2,97 | 0,40 | 0,97 | 0,14 | 648,6 | 246,3 |
| Surénale | 25,12 | 2,72 | 8,12 | 2,67 | 783,7 | 240,6 |
| Séminales | 0,35 | 0,28 | 0,30 | 0,24 | 166,7 | 47,5 |
| Cerveau | 0,10 | 0,00 | 0,10 | 0,01 | 160,4 | 25,8 |
| Coeur | 6,05 | 0,99 | 0,97 | 0,07 | 178,1 | 102,8 |
| Muscle | 0,63 | 0,08 | 0,27 | 0,04 | 131,7 | 28,7 |
| Pancréas | 0,99 | 0,18 | 0,49 | 0,11 | 266,2 | 82,1 |
| Salivaires | 2,07 | 0,21 | 1,54 | 0,20 | 489,7 | 53,1 |

Tableau 21

| | 72h | | | | | |
|---|---|---|---|---|---|---|
| | [3H]CHE | | [14C]CO | | DiD | |
| | Mean | STD | Mean | STD | MEAN | STD |
| Foie | 24,97 | 2,51 | 0,61 | 0,08 | 648,0 | 103,1 |
| Intestin | 3,47 | 0,62 | 0,49 | 0,12 | 267,8 | 33,8 |
| Poumon | 3,04 | 0,41 | 0,67 | 0,06 | 286,6 | 104,7 |
| Rate | 6,40 | 2,62 | 0,48 | 0,15 | 278,0 | 68,4 |
| Rein | 1,82 | 0,39 | 0,78 | 0,07 | 160,3 | 23,1 |
| Gras | 0,70 | 0,12 | 1,87 | 0,55 | 78,0 | 11,3 |
| Testicule | 4,34 | 1,62 | 1,43 | 0,47 | 481,2 | 105,0 |
| Surénale | 46,41 | 8,98 | 14,65 | 0,95 | 717,3 | 268,8 |
| Séminales | 0,58 | 0,03 | 0,47 | 0,03 | 74,4 | 1,3 |
| Cerveau | 0,11 | 0,02 | 0,10 | 0,01 | 14,8 | 8,6 |
| Coeur | 7,83 | 3,47 | 0,88 | 0,35 | 142,3 | 43,0 |
| Muscle | 0,49 | 0,20 | 0,35 | 0,21 | 109,2 | 23,0 |
| Pancréas | 1,12 | 0,36 | 0,63 | 0,19 | 160,6 | 22,1 |
| Salivaires | 2,36 | 0,42 | 1,38 | 0,66 | 430,2 | 8,2 |

La comparaison des valeurs des tableaux 6 à 14 et des tableaux 15 à 21 montre que la biodistribution *in vivo* entre les femelles et les mâles est identique concernant l'accumulation dans le foie et les surrénales.

Exemple 2.2.: Résultat de biodistribution de la formulation de l'exemple 1.2.

Le tableau 22 fournit les résultats de biodistribution du DiD exprimé en intensité de fluorescence selon l'organe de la souris 4 heures après injection à une souris femelle FVB de la nanoémulsion de l'exemple 1.2.1. ou 1.2.2. comprenant le traceur DiD et le stéarate de cholestéryle (CHST) comme composé de formule (I).

tableau 22

| % en stéarate de cholestéryle dans la phase dispersée | 0% (composé exempte de composé de formule (I)) (expl comparatif) | | 0,6%CHST (exemple 1.2.1.) | | 2%CHST (exemple 1.2.2.) | |
|---|---|---|---|---|---|---|
| | MEAN | STD | MEAN | STD | MEAN | STD |
| Cerveau | 249,8 | 62,0 | 303 | 86 | 280 | 79 |
| Coeur | 619,6 | 69,1 | 549 | 134 | 624 | 38 |

(suite)

| % en stéarate de cholestéryle dans la phase dispersée | 0% (composé exempte de composé de formule (I)) (expl comparatif) | | 0,6%CHST (exemple 1.2.1.) | | 2%CHST (exemple 1.2.2.) | |
|---|---|---|---|---|---|---|
| Foie | 1585,5 | 270,9 | 1598 | 294 | 1992 | 305 |
| Gras | 299,4 | 52,9 | 426 | 166 | 342 | 70 |
| Intestin | 532,4 | 68,1 | 627 | 249 | 548 | 92 |
| Muscle | 198,9 | 22,7 | 235 | 87 | 136 | 43 |
| Ovaire | 1286,6 | 47,2 | 2440 | 491 | 2671 | 659 |
| Pancréas | 353,8 | 89,9 | 472 | 120 | 284 | 7 |
| Poumon | 602,8 | 115,5 | 960 | 213 | 878 | 123 |
| Rate | 730,2 | 142,5 | 737 | 99 | 669 | 48 |
| Rein | 796,8 | 106,8 | 886 | 185 | 747 | 8 |
| Salivaire | 707,7 | 258,7 | 404 | 181 | 320 | 63 |
| Surrénales | 1780,2 | 560,2 | 1498 | 527 | 1476 | 899 |
| Utérus | 1379,6 | 680,5 | 1177 | 45 | 1165 | 534 |

Comme dans l'exemple 2.2., une accumulation est observée dans le foie (zone de métabolisation des nanogouttelettes lipidiques) ainsi que dans les organes stéroïdiens (ovaires et surrénales), organes de synthèse des hormones stéroïdiennes. Ces mêmes zones de synthèse des hormones stéroïdiennes se retrouvent dans les tissus cancéreux des cancers hormonodépendants, tels que par exemple certains cancers du sein ou de la prostate.

**EXEMPLE 3 : Comparaison des formulations selon l'invention et de formulations comprenant du cholestérol à la place du composé de formule (I)**

*** Tests de stabilité**

Des tests de stabilité à 4°C ont été effectués pour des formulations comprenant ou non un agent de diagnostic (DiD) et comprenant soit du stéarate de cholestéryle (composé de formule (I)) (0,6%, 2% et 3% en poids par rapport au poids de la phase dispersée), soit du cholestérol (0,6%, 2%, 5% ou 10% en poids par rapport au poids de la phase dispersée) à la place du composé de formule (I).

En l'absence d'agent de diagnostic, au-delà de 90 jours, on a observé des augmentations de la taille des gouttelettes de phase dispersée et de l'indice de polydispersité (DPI) pour les formulations comprenant 2%, 5% et 10% de cholestérol par rapport au poids de la phase dispersée, ce qui indique un manque de stabilité de ces formulations. Par contre, la taille des gouttelettes de phase dispersée et l'indice de polydispersité des formulations selon l'invention restent constants au-delà de 150 jours, ce qui indique que ces formulations sont stables.

En présence de l'agent de diagnostic, pour la formulation comprenant du cholestérol, on a observé que plus le taux de cholestérol augmente, plus le taux d'encapsulation de l'agent de diagnostic diminue. Là encore, une déstabilisation de ces formulations au-delà de 90 jours à 4°C a été observée. Par contre, aucune différence d'encapsulation du DiD et aucun problème de stabilité n'a été observée pour les formulations comprenant du stéarate de cholestéryle (formulations selon l'invention).

Ainsi, une formulation comprenant plus de 0,6% en poids de cholestérol par rapport au poids de la phase dispersée n'est pas stable au-delà de 90 jours à 4°C, alors que les formulations selon l'invention comprenant un composé de formule (I) sont stables au-delà de 150 jours pour toutes les concentrations testées et permettent une meilleure encapsulation de l'agent de diagnostic que des formulations comprenant du cholestérol.

Sans vouloir être lié à une théorie particulière, il semble que le cholestérol se situe à l'interface des gouttelettes de phase lipidique et les déstabilise, alors que les composés de formule (I), qui sont plus lipophiles que le cholestérol grâce au groupe R, se situent au coeur des gouttelettes et n'affectent pas la stabilité de la nanoémulsion.

*** Test de biodistribution *in vivo* 4 heures après injection à une souris femelle FVB**

Les tableaux 23 à 28 fournissent les résultats de biodistribution (intensité de fluorescence selon l'organe de la souris) 4 heures après injection à une souris femelle FVB de nanoémulsions comprenant du DiD (traceur fluorescent) et :

- exempte de cholestérol ou d'un des ses dérivés (nanoémulsion témoin),

- comprenant du cholestérol (nanoémulsions comparatives), ou
- comprenant un composé de formule (I) (nanoémulsions selon l'invention, selon les exemples 1.1., 1.2.1. ou 1.2.2.).

Tableau 23 : intensité de fluorescence selon l'organe de la souris 4 heures après injection à une souris femelle FVB d'une nanoémulsion comprenant du DiD (traceur) et 0 (témoin), 0,6%, 2%, 5% ou 10% de cholestérol par rapport au poids de la phase dispersée et (nanoémulsions comparatives).

| | Cholestérol - intensité de fluorescence - 4h post injection souris femelle FVB | | | | | | | | | |
|---|---|---|---|---|---|---|---|---|---|---|
| % en cholestérol dans la phase dispersée | 0 (témoin) | | 0,6 (comparatif) | | 2 (comparatif) | | 5 (comparatif) | | 10 (comparatif) | |
| | MEAN | STD | MEAN | STD | MEAN | STD | MEAN | STD | MEAN | STD |
| Cerveau | 249,8 | 62,0 | 116,6 | 44,7 | 288,9 | 12,5 | 308,0 | 132,5 | 149,3 | 79,4 |
| Coeur | 619,6 | 69,1 | 419,3 | 73,7 | 493,2 | 63,4 | 273,9 | 31,9 | 291,2 | 137,2 |
| Foie | 1585,5 | 270,9 | 1146,5 | 129,9 | 1392,6 | 262,6 | 1487,4 | 17,0 | 1459,8 | 670,9 |
| Gras | 299,4 | 52,9 | 355,5 | 98,5 | 257,6 | 31,7 | 237,1 | 60,1 | 261,3 | 79,3 |
| Intestin | 532,4 | 68,1 | 651,3 | 98,8 | 612,7 | 72,1 | 559,3 | 114,2 | 467,6 | 219,1 |
| Muscle | 198,9 | 22,7 | 140,3 | 43,0 | 207,9 | 45,0 | 301,0 | 263,2 | 120,7 | 71,2 |
| Ovaire | 1286,6 | 47,2 | 718,4 | 230,7 | 1427,5 | 472,0 | 1540,8 | 247,9 | 1659,4 | 702,6 |
| Pancréas | 353,8 | 89,9 | 260,0 | 35,2 | 332,7 | 12,1 | 270,5 | 88,9 | 202,3 | 147,8 |
| Poumon | 602,8 | 115,5 | 561,9 | 56,5 | 483,9 | 76,7 | 385,9 | 134,5 | 660,1 | 236,5 |
| Rate | 730,2 | 142,5 | 632,0 | 57,6 | 700,6 | 117,2 | 747,4 | 185,9 | 467,6 | 185,0 |
| Rein | 796,8 | 106,8 | 647,7 | 129,9 | 657,9 | 77,8 | 546,1 | 48,9 | 402,9 | 257,9 |
| Salivaire | 707,7 | 258,7 | 209,6 | 170,6 | 368,9 | 54,6 | 240,9 | 144,0 | 111,6 | 204,8 |
| Surrénales | 1780,2 | 560,2 | 1316,0 | 213,9 | 941,0 | 126,1 | 1039,5 | 558,4 | 1445,5 | 627,3 |
| Utérus | 1379,6 | 680,5 | 842,4 | 173,6 | 1016,1 | 303,0 | 1122,6 | 603,6 | 497,2 | 463,1 |

Tableau 24 : rapport de l'intensité de fluorescence dans un organe de la souris sur l'intensité de fluorescence dans les muscles 4 heures après injection à une souris femelle FVB d'une nanoémulsion comprenant du DiD (traceur) et 0 (témoin), 0,6%, 2%, 5% ou 10% de cholestérol par rapport au poids de la phase dispersée et (nanoémulsions comparatives).

| | Cholestérol - rapport organe / muscle - 4h post injection souris femelle FVB | | | | | | | | | |
|---|---|---|---|---|---|---|---|---|---|---|
| % en cholestérol dans la phase dispersée | 0 (témoin) | | 0,6 (comparatif) | | 2 (comparatif) | | 5 (comparatif) | | 10 (comparatif) | |
| | MEAN | STD | MEAN | STD | MEAN | STD | MEAN | STD | MEAN | STD |
| Cerveau | 1,2 | 0,3 | 0,8 | 0,1 | 1,4 | 0,3 | 2,0 | 0,8 | 1,3 | 0,1 |
| Coeur | 3,1 | 0,3 | 3,3 | 1,7 | 2,5 | 0,8 | 1,8 | 0,1 | 2,6 | 0,6 |
| Foie | 8,3 | 1,2 | 8,9 | 3,8 | 7,0 | 2,4 | 10,1 | 1,5 | 12,8 | 1,8 |
| Gras | 1,5 | 0,2 | 2,8 | 1,3 | 1,3 | 0,2 | 1,6 | 0,2 | 2,8 | 2,0 |
| Intestin | 2,8 | 0,3 | 4,9 | 1,4 | 3,1 | 0,8 | 3,9 | 1,3 | 4,1 | 0,6 |
| Muscle | 1,0 | 0,0 | 1,0 | 0,0 | 1,0 | 0,0 | 1,0 | 0,0 | 1,0 | 0,0 |
| Ovaire | 6,7 | 0,7 | 4,4 | 1,8 | 6,8 | 1,4 | 10,6 | 3,0 | 14,8 | 2,7 |
| Pancréas | 1,8 | 0,4 | 2,0 | 0,9 | 1,7 | 0,5 | 1,8 | 0,6 | 1,7 | 0,5 |
| Poumon | 3,2 | 0,6 | 4,2 | 1,0 | 2,5 | 1,0 | 2,5 | 0,5 | 6,1 | 1,6 |
| Rate | 3,8 | 0,6 | 4,8 | 1,3 | 3,5 | 0,7 | 5,0 | 1,0 | 4,2 | 0,9 |
| Rein | 4,1 | 0,4 | 5,2 | 3,0 | 3,2 | 0,6 | 3,7 | 0,3 | 3,3 | 0,2 |

(suite)

| | Cholestérol - rapport organe / muscle - 4h post injection souris femelle FVB | | | | | | | | | |
|---|---|---|---|---|---|---|---|---|---|---|
| % en cholestérol dans la phase dispersée | 0 (témoin) | | 0,6 (comparatif) | | 2 (comparatif) | | 5 (comparatif) | | 10 (comparatif) | |
| | MEAN | STD | MEAN | STD | MEAN | STD | MEAN | STD | MEAN | STD |
| Salivaire | 3,8 | 1,4 | 1,3 | 1,0 | 1,8 | 0,3 | 1,6 | 1,0 | 0,5 | 1,1 |
| Surrénales | 10,1 | 4,4 | 12,4 | 6,8 | 4,6 | 0,4 | 6,7 | 2,6 | 12,8 | 2,2 |
| Utérus | 7,7 | 3,7 | 6,3 | 1,5 | 4,8 | 0,7 | 7,2 | 2,8 | 3,3 | 2,3 |

Tableau 25 : intensité de fluorescence selon l'organe de la souris 4 heures après injection à une souris femelle FVB d'une nanoémulsion comprenant du DiD (traceur) et 0 (témoin), 0,6%, 2%, 5% ou 10% de cholestérol par rapport au poids de la phase dispersée et (nanoémulsions comparatives).

| | Cholestérol (CH) ou cholestéryl stéarate (CHST) - intensité de fluorescence - 4h post injection souris femelle FVB | | | | | | | | | |
|---|---|---|---|---|---|---|---|---|---|---|
| % en CH ou en CHST dans la phase dispersée | 0% CH (témoin) | | 0,6% (comparatif) | | 2% CH (comparatif) | | 0,6%CHST (exemple 1.2.1.) | | 2%CHST (exemple 1.2.2.) | |
| | MEAN | STD | MEAN | STD | MEAN | STD | MEAN | STD | MEAN | STD |
| Cerveau | 249,8 | 62,0 | 116,6 | 44,7 | 288,9 | 12,5 | 303 | 86 | 280 | 79 |
| Coeur | 619,6 | 69,1 | 419,3 | 73,7 | 493,2 | 63,4 | 549 | 134 | 624 | 38 |
| Foie | 1585,5 | 270,9 | 1146,5 | 129,9 | 1392,6 | 262,6 | 1598 | 294 | 1992 | 305 |
| Gras | 299,4 | 52,9 | 355,5 | 98,5 | 257,6 | 31,7 | 426 | 166 | 342 | 70 |
| Intestin | 532,4 | 68,1 | 651,3 | 98,8 | 612,7 | 72,1 | 627 | 249 | 548 | 92 |
| Muscle | 198,9 | 22,7 | 140,3 | 43,0 | 207,9 | 45,0 | 235 | 87 | 136 | 43 |
| Ovaire | 1286,6 | 47,2 | 718,4 | 230,7 | 1427,5 | 472,0 | 2440 | 491 | 2671 | 659 |
| Pancréas | 353,8 | 89,9 | 260,0 | 35,2 | 332,7 | 12,1 | 472 | 120 | 284 | 7 |
| Poumon | 602,8 | 115,5 | 561,9 | 56,5 | 483,9 | 76,7 | 960 | 213 | 878 | 123 |
| Rate | 730,2 | 142,5 | 632,0 | 57,6 | 700,6 | 117,2 | 737 | 99 | 669 | 48 |
| Rein | 796,8 | 106,8 | 647,7 | 129,9 | 657,9 | 77,8 | 886 | 185 | 747 | 8 |
| Salivaire | 707,7 | 258,7 | 209,6 | 170,6 | 368,9 | 54,6 | 404 | 181 | 320 | 63 |
| Surrénales | 1780,2 | 560,2 | 1316,0 | 213,9 | 941,0 | 126,1 | 1498 | 527 | 1476 | 899 |
| Utérus | 1379,6 | 680,5 | 842,4 | 173,6 | 1016,1 | 303,0 | 1177 | 45 | 1165 | 534 |

tableau 26 : rapport de l'intensité de fluorescence dans un organe de la souris sur l'intensité de fluorescence dans les muscles 4 heures après injection à une souris femelle FVB d'une nanoémulsion comprenant du DiD (traceur) et 0 (témoin), 0,6% ou 2% de cholestérol par rapport au poids de la phase dispersée (nanoémulsions comparatives) ou 0,6% ou 2% de stéarate de cholestéryle par rapport au poids de la phase dispersée (nanoémulsions selon l'invention).

| | Cholestérol (CH) ou cholestéryl stéarate (CHST)- rapport organe / muscle - 4h post injection souris femelle FVB | | | | | | | | | |
|---|---|---|---|---|---|---|---|---|---|---|
| % en CH ou en CHST dans la phase dispersée | 0 % (témoin) | | 0,6 % CH (comparatif) | | 2% CH (comparatif) | | 0,6%CHST (exemple 1.2.1.) | | 2%CHST (exemple 1.2.1.) | |
| | MEAN | STD | MEAN | STD | MEAN | STD | MEAN | STD | MEAN | STD |
| Cerveau | 1,2 | 0,3 | 0,8 | 0,1 | 1,4 | 0,3 | 1,3 | 0,2 | 2,1 | 0,1 |

(suite)

| | Cholestérol (CH) ou cholestéryl stéarate (CHST)- rapport organe / muscle - 4h post injection souris femelle FVB | | | | | | | | | |
|---|---|---|---|---|---|---|---|---|---|---|
| % en CH ou en CHST dans la phase dispersée | 0 % (témoin) | | 0,6 % CH (comparatif) | | 2% CH (comparatif) | | 0,6%CHST (exemple 1.2.1.) | | 2%CHST (exemple 1.2.1.) | |
| | MEAN | STD | MEAN | STD | MEAN | STD | MEAN | STD | MEAN | STD |
| Coeur | 3,1 | 0,3 | 3,3 | 1,7 | 2,5 | 0,8 | 2,4 | 0,4 | 4,8 | 1,2 |
| Foie | 8,3 | 1,2 | 8,9 | 3,8 | 7,0 | 2,4 | 7,3 | 2,6 | 15,1 | 2,6 |
| Gras | 1,5 | 0,2 | 2,8 | 1,3 | 1,3 | 0,2 | 1,8 | 0,3 | 2,7 | 1,4 |
| Intestin | 2,8 | 0,3 | 4,9 | 1,4 | 3,1 | 0,8 | 2,7 | 0,1 | 4,4 | 2,1 |
| Muscle | 1,0 | 0,0 | 1,0 | 0,0 | 1,0 | 0,0 | 1,0 | 0,0 | 1,0 | 0,0 |
| Ovaire | 6,7 | 0,7 | 4,4 | 1,8 | 6,8 | 1,4 | 10,9 | 3,0 | 19,9 | 1,5 |
| Pancréas | 1,8 | 0,4 | 2,0 | 0,9 | 1,7 | 0,5 | 2,3 | 1,2 | 2,2 | 0,6 |
| Poumon | 3,2 | 0,6 | 4,2 | 1,0 | 2,5 | 1,0 | 4,4 | 1,9 | 7,0 | 3,1 |
| Rate | 3,8 | 0,6 | 4,8 | 1,3 | 3,5 | 0,7 | 3,4 | 1,2 | 5,1 | 1,3 |
| Rein | 4,1 | 0,4 | 5,2 | 3,0 | 3,2 | 0,6 | 4,1 | 1,4 | 5,8 | 1,9 |
| Salivaire | 3,8 | 1,4 | 1,3 | 1,0 | 1,8 | 0,3 | 1,7 | 0,1 | 2,6 | 1,3 |
| Surrénales | 10,1 | 4,4 | 12,4 | 6,8 | 4,6 | 0,4 | 6,5 | 1,6 | 10,3 | 3,3 |
| Utérus | 7,7 | 3,7 | 6,3 | 1,5 | 4,8 | 0,7 | 7,6 | 2,2 | 7,6 | 2,2 |

tableau 27 : intensité de fluorescence selon l'organe de la souris 4 heures après injection à une souris femelle FVB d'une nanoémulsion comprenant du DiD (traceur) et 0 (témoin), 0,6% ou 2% de stéarate de cholestéryle par rapport au poids de la phase dispersée (nanoémulsion selon l'invention) ou 0,6% de l'ensemble ([3H]CHE / [14C]CO) par rapport au poids de la phase dispersée (nanoémulsions selon l'invention).

| | [3H]CHE et [14C]CO ou cholestéryl stéarate - intensité de fluorescence - 4h post injection souris femelle FVB | | | | | | | |
|---|---|---|---|---|---|---|---|---|
| % en dérivé de cholestéryle dans la phase dispersée | 0,45% de [3H]CHE et 0,15% de [14C]CO (exemple 1.1.) | | 0 (témoin) | | 0,6%CHST (exemple 1.2.1.) | | 2%CHST (exemple 1.2.2.) | |
| | MEAN | STD | MEAN | STD | MEAN | STD | MEAN | STD |
| Cerveau | 279 | 74 | 249,8 | 62,0 | 303 | 86 | 280 | 79 |
| Coeur | 550 | 87 | 619,6 | 69,1 | 549 | 134 | 624 | 38 |
| Foie | 1845 | 245 | 1585,5 | 270,9 | 1598 | 294 | 1992 | 305 |
| Gras | 522 | 311 | 299,4 | 52,9 | 426 | 166 | 342 | 70 |
| Intestin | 974 | 152 | 532,4 | 68,1 | 627 | 249 | 548 | 92 |
| Muscle | 236 | 64 | 198,9 | 22,7 | 235 | 87 | 136 | 43 |
| Ovaire | 2781 | 181 | 1286,6 | 47,2 | 2440 | 491 | 2671 | 659 |
| Pancréas | 527 | 138 | 353,8 | 89,9 | 472 | 120 | 284 | 7 |
| Poumon | 955 | 19 | 602,8 | 115,5 | 960 | 213 | 878 | 123 |
| Rate | 614 | 108 | 730,2 | 142,5 | 737 | 99 | 669 | 48 |
| Rein | 925 | 81 | 796,8 | 106,8 | 886 | 185 | 747 | 8 |
| Salivaire | 516 | 114 | 707,7 | 258,7 | 404 | 181 | 320 | 63 |
| Surrénales | 1754 | 385 | 1780,2 | 560,2 | 1498 | 527 | 1476 | 899 |
| Utérus | 712 | 538 | 1379,6 | 680,5 | 1177 | 45 | 1165 | 534 |

tableau 28 : rapport de l'intensité de fluorescence dans un organe de la souris sur l'intensité de fluorescence dans les muscles 4 heures après injection à une souris femelle FVB d'une nanoémulsion comprenant du DiD (traceur) et 0 (témoin), 0,6% ou 2% de stéarate de cholestéryle par rapport au poids de la phase dispersée (nanoémulsions selon l'invention) ou 0,6% de l'ensemble ([3H]CHE / [14C]CO) par rapport au poids de la phase dispersée (nanoémulsion selon l'invention).

| | [3H]CHE et [14C]CO ou cholestéryl stéarate - rapport organe / muscle - 4h post injection souris femelle FVB | | | | | | | |
|---|---|---|---|---|---|---|---|---|
| % en cholestérol dans la phase dispersée | 0,45% de [3H]CHE et 0,15% de [14C]CO (exemple 1.1.) | | 0 (témoin) | | 0,6%CHST (exemple 1.2.1.) | | 2%CHST (exemple 1.2.2.) | |
| | MEAN | STD | MEAN | STD | MEAN | STD | MEAN | STD |
| Cerveau | 1,2 | 0,1 | 1,2 | 0,3 | 1,3 | 0,2 | 2,1 | 0,1 |
| Coeur | 2,4 | 0,6 | 3,1 | 0,3 | 2,4 | 0,4 | 4,8 | 1,2 |
| Foie | 8,1 | 1,4 | 8,3 | 1,2 | 7,3 | 2,6 | 15,1 | 2,6 |
| Gras | 2,1 | 0,8 | 1,5 | 0,2 | 1,8 | 0,3 | 2,7 | 1,4 |
| Intestin | 4,5 | 1,8 | 2,8 | 0,3 | 2,7 | 0,1 | 4,4 | 2,1 |
| Muscle | 1,0 | 0,0 | 1,0 | 0,0 | 1,0 | 0,0 | 1,0 | 0,0 |
| Ovaire | 12,3 | 2,8 | 6,7 | 0,7 | 10,9 | 3,0 | 19,9 | 1,5 |
| Pancréas | 2,3 | 0,4 | 1,8 | 0,4 | 2,3 | 1,2 | 2,2 | 0,6 |
| Poumon | 4,3 | 1,3 | 3,2 | 0,6 | 4,4 | 1,9 | 7,0 | 3,1 |
| Rate | 2,7 | 0,3 | 3,8 | 0,6 | 3,4 | 1,2 | 5,1 | 1,3 |
| Rein | 4,1 | 0,9 | 4,1 | 0,4 | 4,1 | 1,4 | 5,8 | 1,9 |
| Salivaire | 2,2 | 0,4 | 3,8 | 1,4 | 1,7 | 0,1 | 2,6 | 1,3 |
| Surrénales | 8,0 | 3,8 | 10,1 | 4,4 | 6,5 | 1,6 | 10,3 | 3,3 |
| Utérus | 3,6 | 3,7 | 7,7 | 3,7 | 7,6 | 2,2 | 7,6 | 2,2 |
| * = triplement marquées | | | | | | | | |

Une augmentation de l'intensité du signal dans les ovaires est observée à des taux d'encapsulation de cholestérol supérieurs à 5% par rapport au poids de la phase dispersée. Il est donc nécessaire d'utiliser au moins 5% en poids de cholestérol par rapport au poids de la phase dispersée pour observer un ciblage plus spécifique envers les organes stéroïdiens qu'envers les autres organes. Il est à noter qu'à des faibles taux de cholestérol, le signal est même inférieur à celui de la formulation exempte de cholestérol (0%).

Or, comme montré ci-dessus dans les tests de stabilité, une formulation d'au moins 5% en cholestérol par rapport au poids de la phase dispersée (proportion nécessaire pour commencer à observer un ciblage spécifique des cancers hormonodépendants) n'est pas stable. Or, la stabilité est une condition essentielle pour une application industrielle.

Par comparaison, les tests de biodistribution *in vivo* à 4h post injection de formulations comprenant 0,6% et 2% en poids de stéarate de cholestéryle par rapport au poids de la phase dispersée et du DiD chez la souris saine FVB (formulation selon l'invention) présentés dans l'exemple 2 ci-dessus montrent que ces formulations ciblent spécifiquement les ovaires, même à faible taux de stéarate de cholestéryle (0,6% en poids par rapport au poids de la phase dispersée).

La comparaison des résultats de biodistribution montre que l'on obtient la même intensité de ciblage dans les ovaires pour des concentrations de 5% en poids en cholestérol par rapport au poids de la phase dispersée et 0,6% en poids de stéarate de cholestéryle par rapport au poids de la phase dispersée, et que le signal avec 2% en poids de stéarate de cholestéryle par rapport au poids de la phase dispersée est supérieur à celui d'une formulation comprenant 10% en poids de cholestérol par rapport au poids de la phase dispersée. Pour une même concentration d'agent de diagnostic, au moins cinq fois plus, généralement huit fois plus de cholestérol que de composé de formule (I) sont nécessaires pour obtenir la même intensité de ciblage des organes stéroïdiens

La formulation selon l'invention comprenant un composé de formule (I) permet donc un ciblage bien plus spécifique des organes stéroïdiens qu'une formulation comprenant du cholestérol.

**EXEMPLE 4 : Comparaison de la spécificité du ciblage entre un cancer hormonodépendant (cancer du sein) et un cancer non hormonodépendant (cancer du cerveau - modèle du gliome)**

Deux types de modèles cancéreux ont été étudiés : le modèle du gliome (cancer du cerveau) et le modèle PyMT (cancer du sein). La cinétique d'accumulation des gouttelettes de la nanoémulsion selon l'exemple 1.1. dans la tumeur d'une souris femelle FVB a été évaluée.

Dans le modèle FVB implanté PyMT (cancer du sein), il a été observé une rapide accumulation du signal dans la zone tumorale. Dès 5h post injection, un rapport tumeur / muscle de 3.5 est observé. Le maximum d'intensité de fluorescence est observé à 21 h post injection avec un rapport de signal tumeur / muscle de 9. Après 21 h, le signal commence à décroitre.

Par contre, l'accumulation dans les zones tumorales est plus lente dans le modèle du gliome. L'intensité maximale de fluorescence est observée 48h post injection versus 21 h post injection pour le modèle FVB PyMT.

Ces résultats montrent que la nanoémulsion cible préférentiellement les cancers hormonodépendants par rapport aux cancers non hormonodépendants.

**EXEMPLE 5 : Influence de la proportion en poids du composé de formule (I) par rapport au poids de phase dispersée sur la possibilité de formuler la nanoémulsion**

Des nanoémulsions ont tentées d'être préparées avec des proportions massiques en stéarate de cholestéryle par rapport au poids total de la phase dispersée supérieures à celles des nanoémulsions selon les exemples 1.2.1. et 1.2.2. décrites ci-dessus (comprenant respectivement 0,6% et 2% de stéarate de cholestéryle par rapport au poids total de la phase dispersée), à savoir avec 3%, 6%, 16% et 33% de stéarate de cholestéryle par rapport au poids total de la phase dispersée.

Tableau 29 : composition de formulations et possibilité de formuler la nanoémulsion selon la proportion massique de stéarate de cholestéryle par rapport au poids total de la phase dispersée.

| | | Matière première | Expl 1.2.1. | Expl 1.2.2. | Expl 5.1. | Expl 5.2. | Expl 5.3. | Expl 5.4. |
|---|---|---|---|---|---|---|---|---|
| phase huileuse | lipide amphiphile | Lipoid | 17 mg | | | | | |
| | lipide solubilisant | Supoccire | 68 mg | | | | | |
| | huile | Huile de soja | 23 mg | | | | | |
| | composé de formule (I) | CHST | 1,2 mg | 4mg | 6mg | 12mg | 40mg | 100mg |
| | %age de masse de composé de formule (I) dans la phase dispersée (en considérant que le co-tensioactif appartient à la phase dispersée) | | 0,60% | 1,96% | 2,91% | 5,66% | 16,67% | 33,33% |
| phase aqueuse | co-tensioactif | PEG | 92 mg | | | | | |
| | solution aqueuse | PBS 1 X | 1800 µL | | | | | |
| Possibilité de formuler la nanoémulsion | | | oui | oui | oui | non | non | non |

Comme illustré dans le tableau 29, il n'a pas été possible de formuler des nanoémulsions pour des proportions en stéarate de cholestéryle par rapport au poids total de la phase dispersée supérieures à 3%.

## Revendications

1. Formulation d'un agent thérapeutique ou de diagnostic sous forme de nanoémulsion, comprenant une phase aqueuse continue et au moins une phase huileuse dispersée, dans laquelle :

   - la phase huileuse comprend :

     - au moins un lipide amphiphile,

- au moins un lipide solubilisant,
- au moins un composé de formule (I) suivante :

$$(R\text{-}L)_n\text{-}A \qquad (I),$$

dans laquelle :

- n représente un nombre entier de 1 à 5, notamment 1 à 2, de préférence 1,
- L représente une liaison simple ou un groupe divalent choisi parmi -O-, -COO-, -OOC-, -CO-NR'-, -NR'-CO-, -S-, -NR'-CO-NR"-, -O-CO-O-, où R' et R" représentent indépendamment H ou un alkyle linéaire ou ramifié de 1 à 20 atomes de carbone et
- R représente un alkyle linéaire ou ramifié comprenant au moins 11 atomes de carbone ou un alcényle linéaire ou ramifié comprenant au moins 11 atomes de carbone, et
- A représente un groupe stéroïdique ou stérolique,

et

- la phase aqueuse comporte au moins un co-tensioactif comprenant au moins une chaîne composée de motifs d'oxyde d'alkylène

la proportion de composé de formule (I) étant comprise de 0,05 à 3% en poids par rapport au poids de la phase huileuse dispersée, le poids de la phase huileuse dispersée incluant le poids du co-tensioactif.

2. Formulation d'un agent thérapeutique ou de diagnostic selon la revendication 1, dans laquelle la phase huileuse comprend au moins un agent thérapeutique pour le traitement de cancers hormonodépendants ou de cancers des organes de synthèse d'hormones stéroïdiennes, ou un agent de diagnostic.

3. Formulation d'un agent thérapeutique ou de diagnostic selon la revendication 1 ou 2, dans laquelle le groupe A est un groupe ayant l'une des formules suivantes :

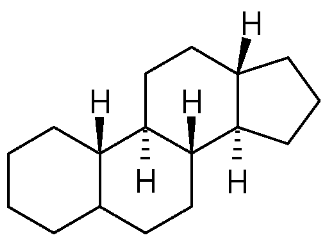

(IIa)

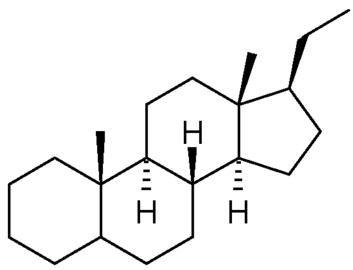

(IIb),

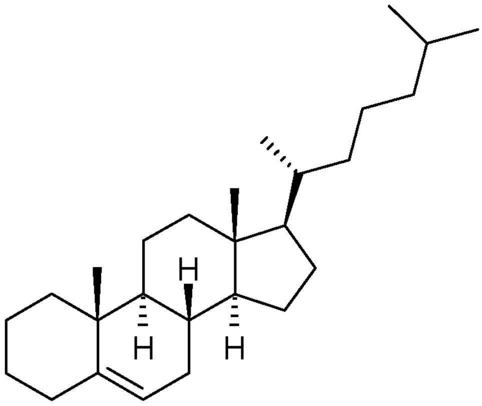

(IIc)

dans lesquelles un ou plusieurs des atomes de carbone est substitué par un groupe -L-R tel que défini dans la revendication 1.

**4.** Formulation d'un agent thérapeutique ou de diagnostic selon l'une quelconque des revendications 1 à 3, dans laquelle L représente -O- ou -COO-, de préférence -COO-.

**5.** Formulation d'un agent thérapeutique ou de diagnostic selon l'une quelconque des revendications 1 à 4, dans laquelle le lipide amphiphile est un phospholipide.

**6.** Formulation d'un agent thérapeutique ou de diagnostic selon l'une quelconque des revendications 1 à 5, dans laquelle le lipide solubilisant comprend au moins un glycéride d'acides gras.

**7.** Formulation d'un agent thérapeutique ou de diagnostic selon la revendication 6, dans laquelle le lipide solubilisant consiste en un mélange de glycérides d'acides gras saturés comportant :

- au moins 10% en poids d'acides gras en C12,
- au moins 5% en poids d'acides gras en C14,
- au moins 5% en poids d'acides gras en C16, et
- au moins 5% en poids d'acides gras en C18.

**8.** Formulation d'un agent thérapeutique ou de diagnostic selon l'une quelconque des revendications 1 à 7, dans laquelle la phase huileuse comporte en outre au moins une huile.

**9.** Formulation d'un agent thérapeutique ou de diagnostic selon l'une quelconque des revendications 1 à 8, dans laquelle le co-tensioactif comporte au moins une chaîne composée de motifs d'oxyde d'éthylène ou d'oxyde d'éthylène et d'oxyde de propylène.

**10.** Formulation d'un agent thérapeutique selon l'une des revendications 1 à 9, comprenant un agent thérapeutique pour le traitement de cancers hormonodépendants ou de cancers des organes de synthèse d'hormones stéroïdiennes choisi parmi :

- les agonistes de la gonadolibérine, tel que l'oestrogène ou les progestatifs,
- les antagonistes de l'aromatase, tels que le Formestane, l'Exemestane, l'Aminoglutethimide, l'Anastrozole, le Fadrozole, le Letrozole, l'acétate de Megestrol, l'acétate de Medroxyprogestérone ou le Mifepristone,
- les anti-oestrogènes, tels que le Tamoxifène ou le Fulvestrant, ou les anti-androgènes, tels que l'acétate d'abiratérone ou l'acétate de cyprotérone.

**11.** Formulation d'un agent de diagnostic selon l'une des revendications 1 à 9, dans laquelle l'agent de diagnostic est un fluorophore lipophile, notamment choisi parmi le vert d'indocyanine, les analogues d'acides gras et les phospholipides fonctionnalisés par un groupement fluorescent, les dérivés amphiphiles de dialkylcarbocyanines tel que le perchlorate de 1,1'-dioctadecyl-3,3,3',3'-tetramethylindodicarbocyanine, les sondes fluorescentes dérivées de sphingolipides, de stéroïdes ou de lipopolysaccharides, les dérivés amphiphiles de cyanines, de rhodamines, de fluorescéines ou de coumarines et le diphénylhexatriène et ses dérivés.

**12.** Procédé de préparation d'une formulation d'un agent thérapeutique ou de diagnostic sous forme de nanoémulsion selon l'une quelconque des revendications 1 à 11, comprenant au moins une phase aqueuse continue et au moins une phase huileuse dispersée, comportant les étapes consistant à :

(i) préparer la phase huileuse comprenant au moins un lipide solubilisant, au moins un lipide amphiphile, éventuellement au moins un agent thérapeutique ou de diagnostic et au moins un composé de formule (I) ;
(ii) préparer une phase aqueuse comprenant un co-tensioactif polyalkoxylé ;
(iii) disperser la phase huileuse dans la phase aqueuse sous l'action d'un cisaillement suffisant pour former une nano-émulsion; et
(iv) récupérer la nano-émulsion ainsi formée.

**13.** Formulation d'un agent thérapeutique selon l'une quelconque des revendications 1 à 10 destinée à être utilisée dans le traitement de cancers hormonodépendants, notamment du sein ou de la prostate, ou de cancers des organes de synthèse d'hormones stéroïdiennes, notamment des ovaires et des surrénales.

**14.** Formulation d'un agent de diagnostic selon l'une quelconque des revendications 1 à 9 ou 11 destinée à être utilisée dans une méthode de diagnostic "in vivo" de cancers hormonodépendants, notamment du sein ou de la prostate, ou de cancers des organes de synthèse d'hormones stéroïdiennes, notamment des ovaires et des surrénales.

**Patentansprüche**

**1.** Formulierung eines therapeutischen oder diagnostischen Mittels in Form einer Nanoemulsion, umfassend eine kontinuierliche wässrige Phase und mindestens eine dispergierte, ölige Phase, wobei:

- die ölige Phase:

  - mindestens ein amphiphiles Lipid,
  - mindestens ein solubilisierendes Lipid,
  - mindestens eine Verbindung der folgenden Formel (I):

$$(R\text{-}L)_n\text{-}A \quad (I),$$

  wobei:

    - n eine ganze Zahl von 1 bis 5, insbesondere 1 bis 2, vorzugsweise 1 darstellt,
    - L eine Einfachbindung oder eine divalente Gruppe ausgewählt aus -O-, -COO-, -OOC-, -CO-NR'-, -NR'-CO-, -S-, -NR'-CO-NR"-, -O-CO-O- darstellt, wobei R' und R" unabhängig H oder ein lineares oder verzweigtes Alkyl mit 1 bis 20 Kohlenstoffatomen darstellen, und
    - R ein lineares oder verzweigtes Alkyl umfassend mindestens 11 Kohlenstoffatome oder ein lineares oder verzweigtes Alkenyl umfassend mindestens 11 Kohlenstoffatome darstellt und
    - A eine Steroid- oder Sterol-Gruppe darstellt,
    umfasst
    und

- die wässrige Phase mindestens ein Cotensid umfassend mindestens eine Kette, die sich aus Alkylenoxid-Motiven zusammensetzt, beinhaltet,
wobei der Anteil der Verbindung der Formel (I) zwischen 0,05 bis 3 Gewichts-% ist, bezogen auf das Gewicht der dispergierten, öligen Phase, wobei das Gewicht der dispergierten, öligen Phase das Gewicht des Cotensids einschließt.

**2.** Formulierung eines therapeutischen oder diagnostischen Mittels gemäß Anspruch 1, wobei die ölige Phase mindestens ein therapeutisches Mittel für die Behandlung hoimonabhängigen Krebses oder Krebses der Organe für die Synthese von Steroidhormonen oder ein diagnostisches Mittel umfasst.

**3.** Formulierung eines therapeutischen oder diagnostischen Mittels gemäß Anspruch 1 oder 2, wobei die Gruppe A

eine Gruppe mit einer der folgenden Formeln ist:

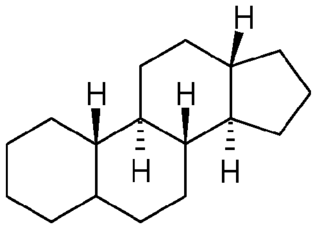

(IIa)

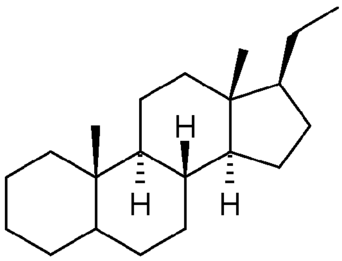

(IIb),

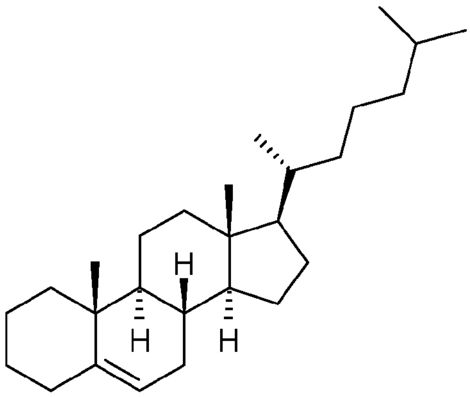

(IIc)

wobei eines oder mehrere der Kohlenstoffatome mit einer Gruppe -L-R substituiert ist, wie in Anspruch 1 definiert.

**4.** Formulierung eines therapeutischen oder diagnostischen Mittels gemäß einem der Ansprüche 1 bis 3, wobei L -O- oder -COO-, vorzugsweise -COO- darstellt.

**5.** Formulierung eines therapeutischen oder diagnostischen Mittels gemäß einem der Ansprüche 1 bis 4, wobei das amphiphile Lipid ein Phospholipid ist.

**6.** Formulierung eines therapeutischen oder diagnostischen Mittels gemäß einem der Ansprüche 1 bis 5, wobei das solubilisierende Lipid mindestens ein Fettsäureglycerid umfasst.

**7.** Formulierung eines therapeutischen oder diagnostischen Mittels gemäß Anspruch 6, wobei das solubilisierende Lipid aus einer Mischung von gesättigten Fettsäureglyceriden beinhaltend:

- mindestens 10 Gewichts-% C12 Fettsäuren,
- mindestens 5 Gewichts-% C14 Fettsäuren,
- mindestens 5 Gewichts-% C16 Fettsäuren und
- mindestens 5 Gewichts-% C18 Fettsäuren

besteht.

**8.** Formulierung eines therapeutischen oder diagnostischen Mittels gemäß einem der Ansprüche 1 bis 7, wobei die ölige Phase des Weiteren mindestens ein Öl beinhaltet.

**9.** Formulierung eines therapeutischen oder diagnostischen Mittels gemäß einem der Ansprüche 1 bis 8, wobei das Cotensid mindestens eine Kette, die aus Ethylenoxid- oder Ethylenoxid- und Propylenoxid-Motiven zusammengesetzt ist, umfasst.

**10.** Formulierung eines therapeutischen Mittels gemäß einem der Ansprüche 1 bis 9, umfassend ein therapeutisches Mittel für die Behandlung hormonabhängigen Krebses oder Krebses der Organe für die Synthese von Steroidhormonen ausgewählt aus:

- Gonadoliberin-Agonisten, wie Östrogen oder Progestinen,
- Aromatase-Antagonisten, wie Formestan, Exemestan, Aminoglutethimid, Anastrozol, Fadrozol, Letrozol, Megestrolacetat, Medroxyprogesteronacetat oder Mifepriston,
- Antiöstrogenen, wie Tamoxifen oder Fulvestrant, oder Antiandrogenen, wie Abirateronacetat oder Cyproteronacetat.

**11.** Formulierung eines diagnostischen Mittels gemäß einem der Ansprüche 1 bis 9, wobei das diagnostische Mittel ein lipophiler Fluorophor ist, insbesondere ausgewählt aus Indocyaningrün, Fettsäureanaloga und Phospholipiden funktionalisiert mit einer fluoreszenten Gruppe, amphiphilen Derivaten von Dialkylcarbocyaninen wie das Perchlorat von 1,1'-Dioctadecyl-3,3,3',3'-tetramethylindodicarbocyanin, fluoreszenten Sonden abgeleitet von Sphingolipiden, Steroiden oder Lipopolysacchariden, amphiphilen Derivaten von Cyaninen, Rhodaminen, Fluoresceinen oder Coumarinen, und Diphenylhexatrien und seinen Derivaten.

**12.** Verfahren zur Herstellung einer Formulierung eines therapeutischen oder diagnostischen Mittels in Form einer Nanoemulsion gemäß einem der Ansprüche 1 bis 11, umfassend mindestens eine kontinuierliche wässrige Phase und mindestens eine dispergierte ölige Phase, beinhaltend die Schritte bestehend aus:

(i) Herstellen der öligen Phase umfassend mindestens ein solubilisierendes Lipid, mindestens ein amphiphiles Lipid, eventuell mindestens ein therapeutisches oder diagnostisches Mittel und mindestens eine Verbindung der Formel (I);
(ii) Herstellen einer wässrigen Phase umfassend ein Polyalkoxyl-Cotensid;
(iii) Dispergieren der öligen Phase in der wässrigen Phase unter Einwirkung einer ausreichenden Scherkraft, um eine Nanoemulsion zu bilden; und
(iv) Gewinnen der so gebildeten Nanoemulsion.

**13.** Formulierung eines therapeutischen Mittels gemäß einem der Ansprüche 1 bis 10, die dafür vorgesehen ist, in der Behandlung hormonabhängigen Krebses, insbesondere der Brust oder der Prostata, oder Krebses der Organe für die Synthese von Steroidhormonen, insbesondere der Eierstöcke und der Nebennieren, verwendet zu werden.

**14.** Formulierung eines diagnostischen Mittels gemäß einem der Ansprüche 1 bis 9 oder 11, die dafür vorgesehen ist, in einem diagnostischen "in vivo" Verfahren hormonabhängigen Krebses, insbesondere der Brust oder der Prostata, oder Krebses der Organe für die Synthese von Steroidhormonen, insbesondere der Eierstöcke und der Nebennieren, verwendet zu werden.

## Claims

**1.** A formulation of a therapeutic or diagnostic agent in the form of a nano-emulsion, comprising a continuous aqueous phase and at least one dispersed oily phase, wherein:

- the oily phase comprises:

  - at least one amphiphilic lipid,
  - at least one solubilizing lipid,
  - at least one compound of the following formula (I):

$$(R\text{-}L)_n\text{-}A \qquad (I),$$

wherein :

- n represents an integer from 1 to 5, notably 1 to 2, preferably 1,
- L represents a simple bond or a divalent group selected from -O- , -COO-, -OOC-, -CO-NR'-, -NR'-CO-, -S-, -NR'-CO-NR"-, -O-CO-O-, wherein R' and R" represent independently H or a linear or branched alkyl with 1 to 20 carbon atoms and
- R represents a linear or branched alkyl comprising at least 11 carbon atoms or a linear or branched alkenyl comprising at least 11 carbon atoms, and
- A represents a steriodic or sterolic group,

and

- the aqueous phase includes at least one co-surfactant comprising at least one chain consisting of alkylene oxide units

the proportion of the compound of formula (I) being from 0.05 to 3% by weight based on the weight of the dispersed oily phase, the weight of the dispersed oily phase including the weight of the co-surfactant.

2. The formulation of a therapeutic or diagnostic agent according to claim 1, wherein the oily phase comprises at least one therapeutic agent for treating hormone-dependent cancers or cancers of organs synthesizing steroidal hormones, or one diagnostic agent.

3. The formulation of a therapeutic or diagnostic agent according to claim 1 or 2, wherein the group A is a group having one of the following formulae:

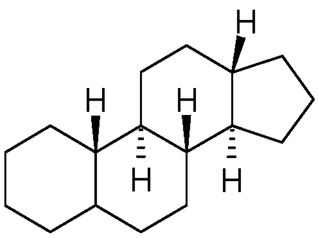

(IIa)

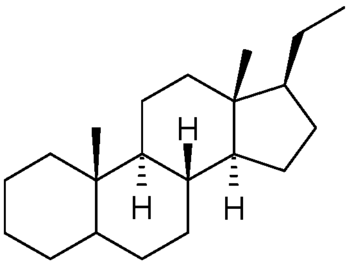

(IIb),

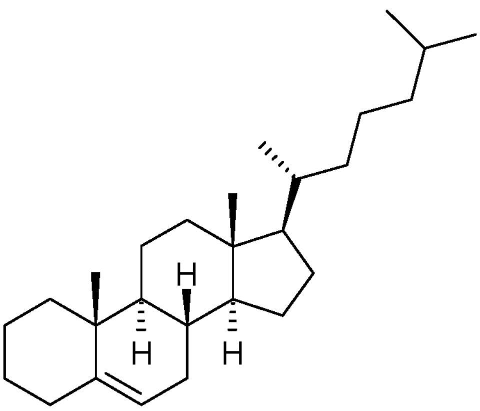

(IIc)

wherein one or several of the carbon atoms are substituted with an -L-R group as defined in claim 1.

**4.** The formulation of a therapeutic or diagnostic agent according to any of claims 1 to 3, wherein L represents -O- or -COO-, preferably -COO-.

**5.** The formulation of a therapeutic or diagnostic agent according to any of claims 1 to 4, wherein the amphiphilic lipid is a phospholipid.

**6.** The formulation of a therapeutic or diagnostic agent according to any of claims 1 to 5, wherein the solubilizing lipid comprises at least one glyceride of fatty acids.

**7.** The formulation of a therapeutic or diagnostic agent according to claim 6, wherein the solubilizing lipid consists in a mixture of saturated fatty acid glycerides including:

- at least 10% by weight of C12 fatty acids,
- at least 5% by weight of C14 fatty acids,
- at least 5% by weight of C16 fatty acids, and
- at least 5% by weight of C18 fatty acids.

**8.** The formulation of a therapeutic or diagnostic agent according to any of claims 1 to 7, wherein the oily phase further includes at least one oil.

**9.** The formulation of a therapeutic or diagnostic agent according to any of claims 1 to 8, wherein the co-surfactant includes at least one chain consisting of ethylene oxide or ethylene oxide and propylene oxide units.

**10.** The formulation of a therapeutic agent according to one of claims 1 to 9, comprising a therapeutic agent for treating hormone-dependent cancers or cancers of organs synthesizing steroidal hormones selected from:

- agonists of gonadotropin releasing hormone such as estrogen or progestins,
- antagonists of aromatase, such as Formestane, Exemestane, Aminoglutethimide, Anastrozole, Fadrozole, Letrozole, Megestrol acetate, Medroxyprogesterone acetate or Mifepristone,
- anti-estrogens, such as Tamoxifene or Fulvestrant, or anti-androgens, such as abiraterone acetate or cyproterone acetate.

**11.** The formulation of a diagnostic agent according to one of claims 1 to 9, wherein the diagnostic agent is a liphophilic fluorophore notably selected from indocyanine green, fatty acid analogs and phospholipids functionalized with a fluorescent group, amphiphilic derivatives of dialkylcarbocyanines such as 1,1'-dioctadecyl-3,3,3',3'-tetramethylindodicarbocyanine perchlorate, fluorescent probes derived from sphingolipids, steroids or lipopolysaccharides, amphiphilic derivatives of cyanines, rhodamines, fluoresceins or cumarins and diphenylhexatriene and its derivatives.

**12.** A method for preparing a formulation of a therapeutic or diagnostic agent in the form of a nano-emulsion according to any of claims 1 to 11, comprising at least one continuous aqueous phase and at least one dispersed oily phase, including the steps:

(i) preparing the oily phase comprising at least one solubilizing lipid, at least one amphiphilic lipid, optionally at least one therapeutic or diagnostic agent and at least one compound of formula (I);
(ii) preparing an aqueous phase comprising a polyalkoxylated co-surfactant;
(iii) dispersing the oily phase in the aqueous phase under the action of sufficient shearing for forming a nano-emulsion; and
(iv) recovering the thereby formed nano-emulsion.

**13.** The formulation of a therapeutic agent according to any of claims 1 to 10 for its use for treating hormone-dependent cancers, notably breast or prostate cancer, or cancers of organs synthesizing steroidal hormones, notably ovaries and adrenal glands.

**14.** Formulation of a diagnostic agent according to any of claims 1 to 9 or 11 for its use in an "in vivo" diagnostic method of hormone-dependent cancers, notably breast or prostate cancer, or cancers of organs synthesizing steroidal hormones, notably ovaries and adrenal glands.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2010018223 A
- WO 2008102065 A


### Littérature non-brevet citée dans la description


- **ALMEIDA et al.** *Int. J. Nanomedicine,* 2010, vol. 5, 679-686
- **C. SOLANS ; P. IZQUIERDO ; J. NOLLA ; N. AZEMAR ; M. J. GARCIA-CELMA.** *Curr Opin Colloid In,* 2005, vol. 10, 102-110
- *Pure&Appl. Chem.,* 1989, vol. 61 (10), 1783-1822
- **MAEDA H ; WU J ; SAWA T ; MATSUMURA Y ; HORI K.** *J Control Release,* 2000, vol. 65, 271-284